(19) Europäisches Patentamt
European Patent Office
Office européen des brevets

(11) Veröffentlichungsnummer: **0 399 159**
**A2**

(12) **EUROPÄISCHE PATENTANMELDUNG**

(21) Anmeldenummer: **90105027.8**

(51) Int. Cl.5: **C07D 221/10, A61K 31/47**

(22) Anmeldetag: **16.03.90**

(30) Priorität: **20.03.89 CH 1028/89**
**21.12.89 CH 4593/89**

(43) Veröffentlichungstag der Anmeldung:
**28.11.90 Patentblatt 90/48**

(84) Benannte Vertragsstaaten:
**AT BE CH DE DK ES FR GB GR IT LI LU NL SE**

(71) Anmelder: **F. HOFFMANN-LA ROCHE AG**
**Postfach 3255**
**CH-4002 Basel(CH)**

(72) Erfinder: **Imhof, René, Dr.**
**Bleumatthöhe 3**
**CH-5264 Gipf-Oberfrick(CH)**
Erfinder: **Keller, Hans Hermann, Dr.**
**Lange Gasse 88**
**CH-4052 Basel(CH)**

(74) Vertreter: **Lederer, Franz, Dr. et al**
**Lederer, Keller & Riederer, Patentanwälte,**
**Lucile-Grahn-Strasse 22**
**D-8000 München 80(DE)**

(54) **Chinolinderivate.**

(57) Die Verbindungen der Formel

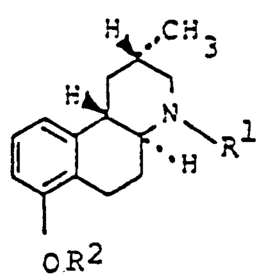

I

worin $R^1$ Wasserstoff, nieder-Alkyl, welches gegebenenfalls durch Hydroxy, nieder-Alkoxy, Cyan, Halogen, nieder-Alkoxycarbonyl, Aminocarbonyl, substituiertes Benzoyl oder substituiertes α-Hydroxybenzyl substituiert ist, nieder-Alkenyl, Cycloalkyl, Cycloalkyl-nieder-alkyl, Heteroaryl-nieder-alkyl oder gegebenenfalls durch Hydroxy, nieder-Alkoxy, nieder-Alkyl oder Halogen substituiertes Aryl-nieder-alkyl und $R^2$ Wasserstoff oder nieder-Alkanoyl bedeuten,
in Form der Racemate und der optischen Antipoden sowie pharmazeutisch verwendbare Säureadditionssalze davon sind aktiv als selektive, präsynaptisch wirkende Dopaminrezeptoragonisten und eignen sich demnach zur Bekämpfung bzw. Verhütung von Erkrankungen des Zentralnervensystems, insbesondere zur Bekämpfung bzw. Verhütung psychotischer Störungen, wie chronischer Schizophrenie. Sie können nach verschiedenen, an sich bekannten Verfahren hergestellt werden.

EP 0 399 159 A2

## Chinolinderivate

Die vorliegende Erfindung betrifft Octahydrobenzo[f]-chinolinderivate. Im speziellen betrifft sie Octahydrobenzo[f]chinoline der allgemeinen Formel

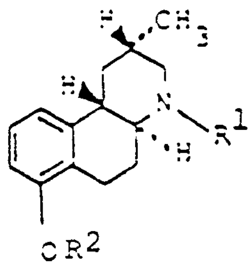

I

worin $R^1$ Wasserstoff, nieder-Alkyl, welches gegebenenfalls durch Hydroxy, nieder-Alkoxy, Cyan. Halogen. nieder-Alkoxycarbonyl. Aminocarbonyl. substituiertes Benzoyl oder substituiertes uHydroxybenzyl substituiert ist. nieder-Alkenyl. Cycloalkyl. Cycloalkylnieder-alkyl. Heteroaryl-nieder-alkyl oder gegebenenfalls durch Hydroxy, nieder-Alkoxy, nieder-Alkyl oder Halogen substituiertes Aryl-nieder-alkyl und $R^2$ Wasserstoff oder nieder-Alkanoyl bedeuten.
in Form der Racemate und der optischen Antipoden sowie pharmazeutisch verwendbare Säureadditionssalze davon.

Im Rahmen der vorliegenden Erfindung wurde überraschenderweise gefunden, dass die Verbindungen der Formel I bei geringer Toxizität interessante und therapeutisch verwertbare pharmakodynamische Eigenschaften aufweisen. In Tierversuchen konnte nämlich gezeigt werden, dass die Verbindungen der obigen Formel I sowie ihre pharmazeutisch verwendbaren Säureadditionssalze selektive. präsynaptisch wirkende Dopaminrezeptoragonisten sind.

Gegenstand der vorliegenden Erfindung sind die Verbindungen der allgemeinen Formel I sowie ihre pharmazeutisch verwendbaren Säureadditionssalze per se und als pharmazeutische Wirkstoffe, Arzneimittel, enthaltend eine Verbindung der allgemeinen Formel I oder ein pharmazeutisch verwendbares Säureadditionssalz davon, die Herstellung solcher Arzneimittel und die Verwendung von Verbindungen der allgemeinen Formel I sowie ihrer pharmazeutisch verwendbaren Säureadditionssalze bei der Bekämpfung bzw. Verhütung von Krankheiten bzw. bei der Verbesserung der Gesundheit. insbesondere bei der Bekämpfung bzw. Verhütung von Erkrankungen des Zentralnervensystems, insbesondere von psychotischen Störungen, wie chronischer Schizophrenie. Gegenstand der vorliegenden Erfindung ist schliesslich ein Verfahren zur Herstellung der Verbindungen der obigen Formel I und ihrer pharmazeutisch verwendbaren Säureadditionssalze sowie die in diesem Verfahren eingesetzten Zwischenprodukte.

Der in der vorliegenden Beschreibung verwendete Ausdruck "nieder-Alkyl" - allein oder in Kombination - bedeutet geradkettige und verzweigte, gesättigte Kohlenwasserstoffreste mit 1-8. vorzugsweise 1-4, Kohlenstoffatomen. wie Methyl, Aethyl. n-Propyl, Isopropyl, n-Butyl, Isobutyl, sec.-Butyl. t-Butyl. Pentyl, Hexyl und dergleichen. Der Ausdruck "nieder-Alkoxyl" bedeutet nieder-Alkyläthergruppen. worin der Ausdruck "nieder-Alkyl" die obige Bedeutung hat, wie Methoxy, Aethoxy. Propoxy, Isopropoxy, Butoxy, Isobutoxy, sec.-Butoxy, t-Butoxy, Pentyloxy, Hexyloxy und dergleichen. Der Ausdruck "Halogen" umfasst die vier Halogene Fluor, Chlor. Brom und Jod. Der Ausdruck "substituiertes Benzoyl" bedeutet einen, vorzugsweise in der 4-Stellung. durch nieder-Alkyl, nieder-Alkoxy oder Halogen monosubstituierten Benzoylrest. In ähnlicher Weise bedeutet der Ausdruck "substituiertes α-Hydroxybenzyl" einen, vorzugsweise in der 4-Stellung. durch nieder-Alkyl, nieder-Alkoxy oder Halogen monosubstituierten α-Hydroxybenzylrest. Der Ausdruck "nieder-Alkenyl" bedeutet geradkettige und verzweigte, ungesättigte Kohlenwasserstoffreste mit 2-8, vorzugsweise 2-4, Kohlenstoffatomen, wie Vinyl, Allyl und dergleichen. Der Ausdruck "Cycloalkyl" bedeutet gesättigte, cyclische Kohlenwasserstoffreste mit 3-8. vorzugsweise 3-6, Kohlenstoffatomen, wie Cyclopropyl. Cyclobutyl, Cyclopentyl, Cyclohexyl und dergleichen. Der Ausdruck "Heteroaryl" bezeichnet einen mono- oder bicyclischen aromatischen Kohlenwasserstoffrest, in welchem ein oder mehrere Kohlenstoffatome durch 1-2 Stickstoffatome und oder ein Sauerstoff- oder Schwefelatom ersetzt sind, wie Pyrrolyl, Furyl, Thienyl, Imidazolyl, Pyrazolyl. Oxazolyl. Thiazolyl, Pyridyl, Pyrazinyl, Pyrimidinyl, Indolyl, Chinolyl, Isochinolyl, Isoxalinyl und dergleichen. Der Ausdruck "Aryl-nieder-alkyl" bezeichnet geradkettige oder verzweigte nieder-Alkylgruppen, worin ein oder mehrere Wasserstoffatome durch Phenyl ersetzt sind, wie

Benzyl, Diphenylmethyl, Trityl, 2-Phenyläthyl, 3-Phenylpropyl. 4-Phenylbutyl und dergleichen. Der Ausdruck "nieder-Alkanoyl" bedeutet den Säurerest einer geradkettigen oder verzweigten Alkansäure mit 1-8, vorzugsweise 1-4, Kohlenstoffatomen, wie Formyl, Acetyl, Propionyl, Butyryl, Valeryl, Isovaleryl und dergleichen.

Der Ausdruck "pharmazeutisch verwendbare Säureadditionssalze" umfasst Salze mit anorganischen und organischen Säuren, wie Salzsäure, Bromwasserstoffsäure, Salpetersäure, Schwefelsäure, Phosphorsäure, Zitronensäure, Ameisensäure. Maleinsäure, Essigsäure, Bernsteinsäure, Weinsäure, Methansulfonsäure, p-Toluolsulfonsäure und dergleichen. Solche Salze können im Hinblick auf den Stand der Technik und unter Berücksichtigung der Natur der in ein Salz zu überführenden Verbindung durch jeden Fachmann ohne weiteres hergestellt werden.

Die Verbindungen der Formel I besitzen in den Positionen 2, 4a und 10b asymmetrische Kohlenstoffatome, liegen aber in der durch die Formel I angegebenen relativen Konfiguration vor, weshalb sie auch nicht in Form von Diastereomeren oder Diastereomerengemischen, sondern nur in Form von Racematen oder optischen Antipoden vorliegen. Die Erfindung umfasst die Racemate und die optischen Antipoden. Racemate können nach üblichen Methoden aufgetrennt werden, z.B. durch Säulenchromatographie, Dünnschichtchromatographie, HPLC, fraktionierte Kristallisation und dergleichen.

Bevorzugte Verbindungen der Formel I sind solche, worin $R^2$ Wasserstoff oder Acetyl, vorzugsweise Wasserstoff, bedeutet.

Weitere bevorzugte Verbindungen der Formel I sind solche, worin $R^1$ Wasserstoff, nieder-Alkyl, welches gegebenenfalls durch Hydroxy, Cyan oder substituiertes Benzoyl substituiert ist. Cycloalkyl, Cycloalkylalkyl oder gegebenenfalls durch Hydroxy substituiertes Aryl-nieder-alkyl bedeutet.

Besonders bevorzugt sind diejenigen Verbindungen, worin $R^1$ Wasserstoff, nieder-Alkyl, welches gegebenenfalls durch Hydroxy, Cyan oder 4-Methoxybenzoyl substituiert ist, wobei der nieder-Alkylrest vorzugsweise Aethyl, Propyl oder Isopropyl ist, Cyclopentyl, Cyclopentyläthyl oder 4-Hydroxyphenäthyl bedeutet.

Aus dem obigen folgt, dass von den Verbindungen der Formel 1 diejenigen ganz besonders bevorzugt sind, worin $R^1$ Wasserstoff, gegebenenfalls durch Hydroxy, Cyan oder 4-Methoxybenzoyl substituiertes Aethyl, Propyl oder Isopropyl, Cyclopentyl, Cyclopentyläthyl oder 4-Hydroxyphenäthyl und $R^2$ Wasserstoff bedeuten.

Ganz speziell bevorzugte Verbindungen der Formel I sind:

rac-1,2,3,4,4aα,5,6,10bβ-Octahydro-2α-methyl-4-propylbenzo[f]chinolin-7-ol,
rac-1,2,3,4,4aα,5,6,10bβ-Octahydro-4-(4-hydroxyphen
äthyl)-2α-methylbenzo[f]chinolin-7-ol,
rac-1,2,3,4,4aα,5,6,10bβ-Octahydro-7-hydroxy-2α-methylbenzo[f]chinolin-4-äthanol,
rac-1,2,3,4,4aα.5,6,10bβ-Octahydro-7-hydroxy-2α-methylbenzo[f]chinolin-4-butyronitril,
rac-1,2,3,4,4aα,5,6,10bβ-Octahydro-4-cyclopentyl-2α-methylbenzo[f]chinolin-7-ol,
rac-1,2,3,4,4aα,5,6,10bβ-Octahydro-4-isopropyl-2α-methylbenzo[f]chinolin-7-ol,
rac-1,2,3,4,4aα,5,6,10bβ-Octahydro-4-cyclopentyläthyl-2α-methylbenzo[f]chinolin-7-ol,
rac-[1,2,3,4,4aα, 5,6,10bβ-Octahydro-7-hydroxy-2α-methylbenzo[f]chinolin-4-yl]-4'-methoxybutyrophenon,
rac-1,2,3,4,4aα,5,6,10bβ-Octahydro-2-α-methylbenzo[f]-chinolin-7-ol,
(-)-1,2,3,4,4aα,5,6,10bβ-Octahydro-4-cyclopentyl-2α-methylbenzo[f]chinolin-7-ol,
(-)-1,2,3,4,4aα,5,6,10bβ-Octahydro-2α-methyl-4-propylbenzo[f]chinolin-7-ol,
(-)-1,2,3,4,4aα,5,6,10bβ-Octahydro-7-hydroxy-2α-methylbenzo[f]chinolin-4-äthanol und
(-)-1,2,3,4,4aα,5,6,10bβ-Octahydro-2α-methylbenzo[f]chinolin-7-ol.

Die Verbindungen der Formel I und deren pharmazeutisch verwendbare Säureadditionssalze lassen sich erfindungsgemäss herstellen, indem man

a) zur Herstellung einer Verbindung der Formel I, worin $R^2$ Wasserstoff bedeutet, in einer Verbindung der allgemeinen Formel

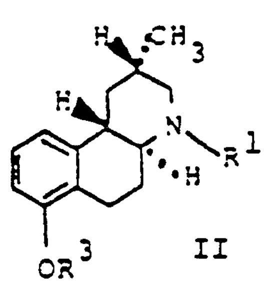

II

worin $R^3$ nieder-Alkyl bedeutet und $R^1$ die oben angegebene Bedeutung besitzt, die Aethergruppe spaltet, oder

b) zur Herstellung einer Verbindung der Formel I, worin $R^2$ Wasserstoff bedeutet, eine Verbindung der allgemeinen Formel

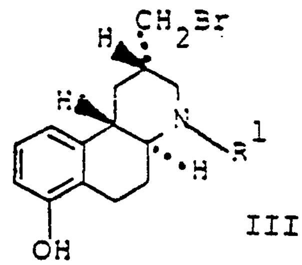

III

worin $R^2$ die oben angegebene Bedeutung besitzt, reduktiv dehalogeniert, oder

c) zur Herstellung einer Verbindung der Formel I, worin $R^1$ nieder-Alkyl, welches gegebenenfalls durch Hydroxy, nieder-Alkoxy, Cyan, Halogen, nieder-Alkoxycarbonyl, Aminocarbonyl, substituiertes Benzoyl oder substituiertes α-Hydroxybenzyl substituiert ist, nieder-Alkenyl, Cycloalkyl, Cycloalkyl-nieder-alkyl, Heteroaryl-nieder-alkyl oder gegebenenfalls durch Hydroxy, nieder-Alkoxy, nieder-Alkyl oder Halogen substituiertes Aryl-nieder-alkyl und $R^2$ Wasserstoff bedeuten, eine Verbindung der Formel I, worin $R^1$ und $R^2$ je Wasserstoff bedeuten, am Stickstoffatom entsprechend substituiert, oder

d) zur Herstellung einer Verbindung der Formel I, worin $R^2$ nieder-Alkanoyl bedeutet, eine Verbindung der Formel I, worin $R^2$ Wasserstoff bedeutet, O-acyliert, oder

e) zur Herstellung einer Verbindung der Formel I, worin $R^1$ durch Hydroxy oder substituiertes α-Hydroxybenzyl substituiertes nieder-Alkyl und $R^2$ Wasserstoff bedeuten, eine Verbindung der Formel I, worin $R^1$ durch nieder-Alkoxycarbonyl oder substituiertes Benzoyl substituiertes nieder-Alkyl und $R^2$ Wasserstoff bedeuten, reduziert, oder

f) zur Herstellung einer Verbindung der Formel I, worin $R^1$ und $R^2$ je Wasserstoff bedeuten, eine Verbindung der Formel I, worin $R^1$ Benzyl und $R^2$ Wasserstoff bedeuten, debenzyliert, und

g) erwünschtenfalls ein erhaltenes Racemat in die optischen Antipoden auftrennt, und oder

h) erwünschtenfalls eine erhaltene Verbindung in ein pharmazeutisch verwendbares Säureadditionssalz überführt.

Die Aetherspaltung einer Verbindung der Formel II gemäss Verfahrensvariante a) erfolgt nach an sich bekannten Methoden. So kann die Aetherspaltung beispielsweise mit Bromwasserstoffsäure bei einer Temperatur zwischen etwa 50° C und der Rückflusstemperatur, vorzugsweise bei der Rückflusstemperatur, oder mit Phosphortribromid in einem unter den RQaktionsbedingungen inerten organischen Lösungsmittel bei einer Temperatur zwischen etwa -10° C und Raumtemperatur, vorzugsweise bei Raumtemperatur, durchgeführt werden. Geeignete organische Lösungsmittel sind chlorierte Kohlenwasserstoffe, wie Methylenchlorid, Chloroform oder Tetrachlorkohlenstoff, Aether, wie Diäthyläther, Tetrahydrofuran oder Dioxan und dergleichen. Das bevorzugte Lösungsmittel ist Methylenchlorid. Es ist klar, dass bei dieser Reaktion eine im Substituenten $R^1$ vorhandene Aethergruppe ebenfalls gespalten wird.

Die reduktive Dehalogenierung einer Verbindung der Formel III gemäss Verfahrensvariante b) erfolgt ebenfalls in bekannter Weise mit Wasserstoff in Gegenwart eines geeigneten Katalysators, z.B. Palladium oder Raney-Nickel. Diese Reaktionsstufe wird zweckmässigerweise in Gegenwart eines inerten organischen Lösungsmittels oder Lösungsmittelgemisches durchgeführt. Geeignete Lösungsmittel sind Alkohole, wie Methanol, Aethanol oder Butanol, Kohlenwasserstoffe, wie Hexan, Benzol, Toluol oder Xylol, Aether, wie Diäthyläther, Tetrahydrofuran oder Dioxan, Alkansäuren, wie Essigsäure, und dergleichen. Die Reaktion wird vorzugsweise bei einer Temperatur zwischen etwa Raumtemperatur und dem Siedepunkt des Reaktionsgemisches, vorzugsweise bei Raumtemperatur, durchgeführt. Der Druck ist nicht kritisch, weshalb aus Zweckmässigkeitsgründen bei Atmosphärendruck gearbeitet wird.

Die Ueberführung einer Verbindung der Formel I, worin $R^1$ und $R^2$ je Wasserstoff bedeuten, in eine entsprechend N-substituierte Verbindung gemäss Verfahrensvariante c) erfolgt ebenfalls nach an sich bekannten Methoden. So kann beispielsweise eine Verbindung der Formel I, worin $R^1$ und $R^2$ je Wasserstoff bedeuten, mit einem entsprechenden Halogenid in Gegenwart eines säurebindenden Mittels in einem unter den Reaktionsbedingungen inerten organischen Lösungsmittel umgesetzt werden. Geeignete säurebindende Mittel sind Basen, wie tertiäre Amine, z.B. Triäthylamin oder Aethyldiisopropylamin, Natriumcarbonat,

Kaliumcarbonat und dergleichen. Beispiele geeigneter Lösungsmittel sind Aethylmethylketon, Aceton, Methylenchlorid, Methanol und dergleichen. Die Reaktion wird zweckmässigerweise bei einer Temperatur zwischen etwa Raumtemperatur und der Rückflusstemperatur des Reaktionsgemisches, vorzugsweise bei der Rückflusstemperatur, durchgeführt.

Die Umsetzung einer Verbindung der Formel I, worin $R^2$ Wasserstoff bedeutet, mit einem Alkanoylierungsmittel gemäss Verfahrensvariante d) erfolgt ebenfalls nach an sich bekannten Methoden. Geeignete Alkanoylierungsmittel sind nieder-Alkansäureanhydride und -halogenide, vorzugsweise -chloride. Die Umsetzung erfolgt in einem unter den Reaktionsbedingungen inerten organischen Lösungsmittel oder Lösungsmittelgemisch bei einer Temperatur zwischen etwa Raumtemperatur und der Rückflusstemperatur des Reaktionsgemisches, vorzugsweise bei etwa Raumtemperatur. Geeignete Lösungsmittel sind aromatische Kohlenwasserstoffe, wie Toluol, chlorierte Kohlenwasserstoffe, wie Methylenchlorid, Acetonitril und dergleichen. Die Reaktion kann in Gegenwart oder Abwesenheit eines Säurebindemittels, wie Natrium- oder Kaliumcarbonat, Pyridin, Triäthylamin und dergleichen, durchgeführt werden.

Die Reduktion einer Verbindung der Formel I, worin $R^1$ durch nieder-Alkoxycarbonyl oder substituiertes Benzoyl substituiertes nieder-Alkyl bedeutet, gemäss Verfahrensvariante e) wird ebenfalls nach an sich bekannten Methoden durchgeführt. So wird das Ausgangsmaterial zweckmässigerweise mit einem komplexen Metallhydrid, wie Lithiumaluminiumhydrid oder Natriumborhydrid und dergleichen, in einem unter den Reaktionsbedingungen inerten organischen Lösungsmittel umgesetzt. Die Reaktionsbedingungen für die Umsetzung hängen vom verwendeten komplexen Metallhydrid ab. So wird beispielsweise bei Verwendung von Lithiumaluminiumhydrid die Umsetzung vorteilhafterweise in einem Lösungsmittel, wie Diäthyläther oder Tetrahydrofuran, bei der Rückflusstemperatur durchgeführt, während die Reduktion mit Natriumborhydrid vorteilhafterweise in einem Lösungsmittel, wie Methanol oder Aethanol und der gleichen, bei etwa Raumtemperatur erfolgt.

Die Debenzylierung gemäss Verfahrensvariante f) erfolgt ebenfalls nach an sich bekannten Methoden. Zweckmässigerweise erfolgt die Debenzylierung durch Hydrogenolyse mit Wasserstoff in Gegenwart eines Katalysators, wie Palladium, bei etwa Raumtemperatur und Atmosphärendruck.

Die Ausgangsstoffe der Formeln II und III sind neu und ebenfalls Gegenstand der vorliegenden Erfindung. Diese Verbindungen können nach an sich bekannten Methoden hergestellt werden, wie sie im nachfolgenden Schema I zusammengegfasst sind.

Schema I

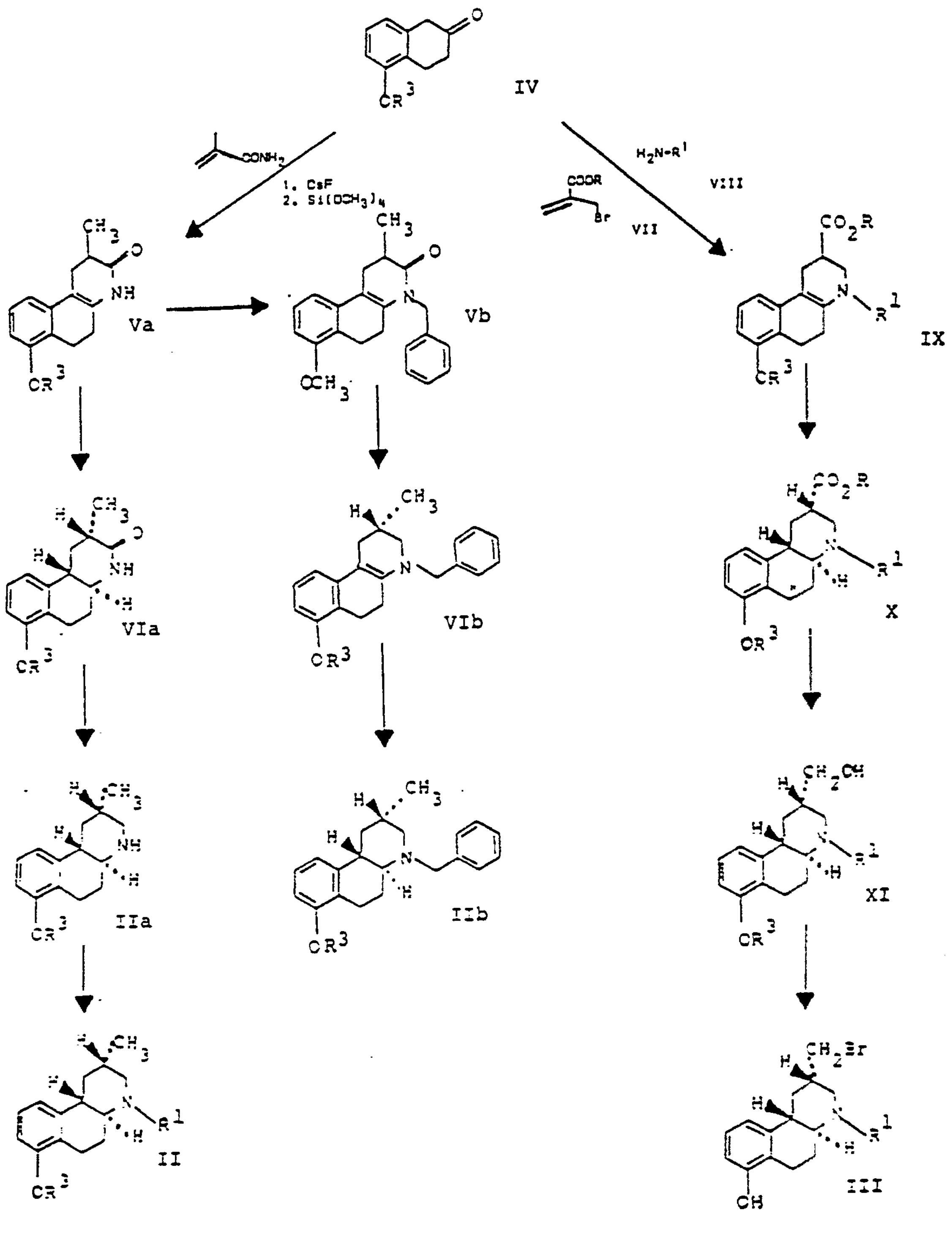


IV, Va, Vb, IX, VIa, VIb, X, IIa, IIb, XI, II, III

R: nieder-Alkyl

Zur Herstellung der Ausgangsstoffe der Formel II kann ein 2-Tetralon der Formel IV zunächst mit Methacrylsäureamid und Cäsiumfluorid und danach mit Tetramethoxysilan umgesetzt werden. Die Umsetzungen können zweckmässigerweise in einem inerten organischen Lösungsmittel wie t-Butylmethyläther und dergleichen bei einer Temperatur zwischen etwa Raumtemperatur und der Rückflusstemperatur des

Reaktionsgemisches durchgeführt werden.

In den nächsten beiden Reaktionsstufen kann eine erhaltene Verbindung der Formel Va ohne Reinigung einer intermediär gebildeten Verbindung der Formel VIa zu einer Verbindung der Formel IIa reduziert werden. Die erste Reduktion zu einer Verbindung der Formel VIa kann in einem unter den Reaktionsbedingungen inerten organischen Lösungsmittel, wie Methylenchlorid und dergleichen, mit Triäthylsilan/Trifluoressigsäure bei etwa Raumtemperatur durchgeführt werden, während die zweite Reduktion zu einer Verbindung der Formel IIa in einem unter den Reaktionsbedingungen inerten organischen Lösungsmittel, wie Tetrahydrofuran und dergleichen, mit Lithiumaluminiumhydrid bei Rückflusstemperatur erfolgt.

Eine erhaltene Verbindung der Formel IIa kann dann unter den für die Verfahrensvariante c) beschriebenen Reaktionsbedingungen in eine entsprechende N-substituierte Verbindung der Formel II übergeführt werden.

Alternativ kann eine erhaltene Verbindung der Formel Va mit Benzylchlorid unter den für die Verfahrensvariante c) beschriebenen Reaktionsbedingungen in eine Verbindung der Formel Vb übergeführt werden, welche dann ebenfalls in zwei Reaktionsstufen, nämlich einer ersten Reduktion mit Lithiumaluminiumhydrid in einem Lösungsmittel, wie Tetrahydrofuran und dergleichen, bei Rückflusstemperatur und einer zweiten Reduktion mit Natriumcyanborhydrid in einem Lösungsmittelgemisch, wie Tetrahydrofuran/Acetonitril oder Tetrahydrofuran/Aethanol, bei Raumtemperatur unter sauren Reduktionsbedingun gen, zu einer Verbindung der Formel IIb reduziert werden kann, wobei auch hier die Reinigung einer intermediär gebildeten Verbindung der Formel VIb nicht notwendig ist.

Zur Herstellung einer Verbindung der Formel III kann ein 2-Tetralon der Formel IV mit einem Amin der Formel VIII und einem 2-(Brommethyl)acrylsäurealkylester der Formel VII zu einer Verbindung der Formel IX umgesetzt werden. Die Umsetzung erfolgt vorzugsweise in einem inerten organischen Lösungsmittel, wie einem aromatischen Kohlenwasserstoff, z.B. Benzol, bei der Rückflusstemperatur, wobei das gebildete Wasser zweckmässigerweise mit einem Wasserabscheider aufgefangen wird.

Eine auf diese Weise erhaltene Verbindung der Formel IX kann dann mit vorzugsweise Natriumcyanborhydrid in einem Alkohol, der der Alkoholkomponente des 2-(Brommethyl)acrylsäurealkylesters entspricht, zu einer Verbindung der Formel X reduziert werden.

Reduktion einer erhaltenen Verbindung der Formel X mit Lithiumaluminiumhydrid in einem inerten organischen Lösungsmittel, wie einem Aether, z.B. Tetrahydrofuran, bei vorzugsweise Raumtemperatur liefert eine Verbindung der Formel XI.

Durch Umsetzen einer Verbindung der Formel XI mit Bromwasserstoffsäure unter den für die Verfahrensvariante a) angegebenen Reaktionsbedingungen erhält man eine Verbindung der Formel III, wobei unter diesen Reaktionsbedingungen gleichzeitig die Hydroxymethylgruppe in 2-Stellung bromiert wird.

Bezüglich der genauen Reaktionsbedingungen für die oben beschriebenen Herstellungsverfahren der Ausgangsstoffe wird auf den Beispielteil verwiesen.

Die Verbindungen der Formeln Va, Vb, VIa, VIb, IX, X und XI sind ebenfalls neu und Gegenstand vorliegender Erfindung. Die Verbindungen der Formel IV sind bekannt oder können in Analogie zur Herstellung der bekannten Verbindungen erhalten werden.

Die Verbindungen der Formel I und ihre pharmazeutisch verwendbaren Säureadditionssalze sind aktiv als selektive, präsynaptisch wirkende Dopaminrezeptoragonisten und eignen sich zur Bekämpfung bzw. Verhütung von Erkrankungen des Zentralnervensystems, insbesondere zur Bekämpfung bzw. Verhütung psychotischer Störungen, wie chronischer Schizophrenie.

Die Wirkung von Dopaminrezeptoragonisten kann experimentell mittels der nachstehend beschriebenen Tests gezeigt werden:

A) Bestimmung der KCl-induzierten Dopamin-Freisetzung aus Gehirnschnitten

Bei dieser von H.H. Keller und M. Da Prada in European Journal of Pharmacology, 119, 247-250 (1985) beschriebenen in vitro-Methode werden isolierte Gehirnschnitte (Striatum) unbehandelter männlicher Ratten mit einem Körpergewicht von 190-210 g (Füllinsdorfer Albino, SPF) mit Tritium-markiertem Dopamin präinkubiert, in kleinen Kammern bei 37° C mit physiologischem Puffer superfundiert, und die spontan und die durch Kaliumchlorid-Depolarisation induzierte an das überströmende Medium abgegebene Radioaktivität gemessen. Die Hemmung der Dopamin-Freisetzung durch den zu testenden präsynaptisch wirkenden Dopaminagonisten wurde in Interaktionsexperimenten indirekt bestimmt, d.h. durch die Hemmung der durch Molindon, einem präsynaptisch wirkenden Dopaminantagonisten, bedingten Erhöhung der durch Kaliumchlorid induzierten Dopamin-Freisetzung. Bezüglich dieser Methode und ihrer Anwendung auf Dopaminagonisten vergleiche H.H. Keller et al., Lisuride and other dopamine agonists, Raven Press, New York, 1983, Seiten 79-87.

B) Bestimmung des Effektes von Dopaminagonisten auf den Homovanillinsäuregehalt im Rattengehirn

Die Testverbindung wird je nach Wasserlöslichkeit in 0,9%iger Natriumchloridlösung (10 ml pro kg + 2 Tropfen Tween 80) gelöst oder suspendiert und männlichen Ratten mit einem Körpergewicht von 140-170 g (Füllindsdorfer Albino, SPF) mit Hilfe einer Schlundsonde verabreicht. 2 Stunden später werden die Tiere ohne Narkose möglichst stressfrei dekapitiert und ihr Gesamthirn bei -80° C eingefroren. Kontrolltiere erhalten nur das Lösungsmittel.

Homovanillinsäure wird (zusammen mit Dopamin, 3,4-Dihydroxyphenylessigsäure, Noradrenalin, 3-Methoxy-4-hydroxy-phenylglycol, 5-Hydroxytryptamin (Serotonin) und 5-Hydroxyindolessigsäure) mittels HPLC (high performance liquid chromatography) und elektrochemischer Detektion nach der von M. Da Prada et al. in Journal of Pharmacology and Experimental Therapeutics 248, 400-414 (1989) beschriebenen Methode bestimmt.

Die in diesen beiden Tests erhaltenen Resultate sind in der folgenden Tabelle zusammengefasst:

Tabelle

| Verbindung | Hemmung der KCl-induzierten Dopamin-Freisetzung aus Ratten Striatum-Schnitten FSC (nMol) | Senkung des Homovanillinsäure (HVA)-Gehaltes im Rattengehirn FSD (mg kg p.o.) |
|---|---|---|
| A | 30 | 0,3 |
| B | 30 | 0,3 |
| C | < 1 | 0,01 |
| D | <10 | 0,1 |
| E | <10 | 0,1 |
| F | 100 | 1,0 |
| G | 100 | 0,3 |

FSC: erste signifikante Konzentration
FSD: erste signifikante Dosis
A: rac-1,2,3,4,4aα,5,6,10bβ-Octahydro-2α-methyl-4-propylbenzo[f]chinolin-7-ol
B: (-)-1,2,3,4,4aα,5,6,10bβ-Octahydro-2α-methyl-4-propylbenzo[f]chinolin-7-ol
C: rac-1,2,3,4,4aα,5,6,10bβ-Octahydro-4-isopropyl-2α-methylbenzo[f]chinolin-7-ol
D: rac-1,2,3,4,4aα,5,6,10bβ-Octahydro-4-cyclopentyl-2α-methylbenzo[f]chinolin-7-ol
E: (-)-1,2,3,4,4aα,5,6,10bβ-Octahydro-4-cyclopentyl-2α-methylbenzo[f]chinolin-7-ol
F: rac-1,2,3,4,4aα,5,6,10bβ-Octahydro-7-hydroxy-2α-methylbenzo[f]chinolin-4-äthanol
G: (-)-1,2,3,4,4aα,5,6,10bβ-Octahydro-7-hydroxy-2α-methylbenzo[f]chinolin-4-äthanol

Die Verbindungen der Formel I sowie deren pharmazeutisch verwendbare Säureadditionssalze können als Heilmittel, z.B. in Form pharmazeutischer Präparate, Verwendung finden. Die pharmazeutischen Präparate können enteral, vorzugsweise oral, z.B. in Form von Tabletten, Lacktabletten, Dragées, Hart- und Weichgelatinekapseln, Lösungen, Emulsionen oder Suspensionen, oder rektal, z.B. in Form von Suppositorien, verabreicht werden. Die Verabreichung kann aber auch parenteral, wie intramuskulär oder intravenös, z.B. in Form von Injektionslösungen, erfolgen.

Zur Herstellung von Tabletten, Lacktabletten, Dragées und Hartgelatinekapseln können die Verbindungen der Formel I sowie ihre pharmazeutisch verwendbaren Säureadditionssalze mit pharmazeutisch inerten, anorganischen oder organischen Excipientien verarbeitet werden. Als solche Excipientien kann man z.B. für Tabletten, Dragées und Hartgelatinekapseln Lactose, Maisstärke oder Derivate davon, Talk, Stearinsäure oder deren Salze etc. verwenden.

Für Weichgelatinekapseln eignen sich als Excipientien z.B. vegetabile Oele, Wachse, Fette, halbfeste und flüssige Polyole etc.

Zur Herstellung von Lösungen und Sirupen eignen sich als Excipientien z.B. Wasser, Polyole, Saccharose, Invertzucker, Glucose etc.

Für Injektionslösungen eignen sich als Excipientien z.B. Wasser, Alkohole, Polyole, Glycerin, vegetabile Oele etc.

Für Suppositorien eignen sich als Excipientien z.B. natürliche oder gehärtete Oele, Wachse, Fette, halbflüssige oder flüssige Polyole etc.

Die pharmazeutischen Präparate können daneben noch Konservierungsmittel, Lösungsvermittler, Viskositäts-erhöhende Stoffe, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutich wertvolle Stoffe enthalten.

Erfindungsgemäss kann man die Verbindungen der allgemeinen Formel I sowie deren pharmazeutisch verwendbare Säureadditionssalze bei der Bekämpfung bzw. Verhütung von psychotischen Störungen, insbesondere chronischer Schizophrenie, verwenden. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 10-500 mg, vorzugsweise etwa 20-300 mg, und bei parenteraler Verabreichung eine solche von 1-50 mg, vorzugsweise 2-25 mg, verteilt auf eine oder mehrere Einzeldosen, angemessen sein, wobei aber die soeben angegebene obere Grenze auch überschritten werden kann, wenn sich dies als angezeigt erweisen sollte. Ueberlicherweise erhalten Kinder die halbe Dosis von Erwachsenen.

Die folgenden Beispiele sollen die vorliegende Erfindung erläutern, sie jedoch in keiner Weise einschränken. Alle Temperaturen sind in Celsius Graden angegeben.

## Beispiel 1

6,0 g (21,9 mMol) rac-1,2,3,4,4aα,5,6,10bβ-Octahydro-2α-methyl-4-propyl -7-methoxybenzo[f]chinolin werden in 240 ml Bromwasserstoffsäure (48%) unter Rühren in einer Argonatmosphäre 1 1/2 Stunden zum Rückfluss erhitzt. Nach dem Abkühlen destilliert man die überschüssige Bromwasserstoff säure unter vermindertem Druck ab und verteilt den Rückstand zwischen Methylenchlorid und gesättigter wässriger Natriumbicarbonatlösung. Die wässrige Phase wird mit 2N Natronlauge auf pH 9 gestellt, und die organische Phase abgetrennt. Nach zweimaliger Extraktion der wässrigen Phase mit Methylenchlorid werden die organischen Extrakte vereinigt, über wasserfreiem Magnesiumsulfat getrocknet und unter vermindertem Druck eingedampft. Der Rückstand wird in 500 ml Methanol gelöst, filtriert und mit Chlorwasserstoff in Aethanol angesäuert. Beim Einengen der Lösung auf 100 ml Volumen kristallisieren 5,56 g (86%) rac-1,2,3,4,4aα,5,6,10bβ-Octahydro-2α-methyl -4-propylbenzo[f]chinolin-7-ol-hydrochlorid aus, Smp. 264-267°.

Das als Ausgangsmaterial verwendete rac-1,2,3,4,4aα-5,6,10bβ-Octahydro-2α-methyl-4-propyl-7-methoxybenzo[f]chinolin wurde wie folgt hergestellt:

a) 100 g (0,568 Mol) 5-Methoxy-2-tetralon (J. Chem. Soc. 1855 (1949), Cornforth and Robinson) in 500 ml t-Butylmethyläther werden mit 53 g (0,642 Mol) Methacrylsäureamid und 85 g (0,568 Mol) Cäsiumfluorid versetzt. Anschliessend werden bei 20° 85 ml (0,568 Mol) Tetramethoxysilan innerhalb von 30 Minuten zugetropft. Nach 1-stündigem Erhitzen zum Rückfluss wird das Gemisch abgekühlt und auf 5 l Wasser gegossen. Extraktion mit total 6 l Methylenchlorid liefert 125,5 g rötliche Kristalle, welche aus Methylenchlorid/Essigester umkristallisiert werden, wobei man 113,3 g (82%) rac-1,2,5,6-Tetrahydro-2-methyl-7-methoxy[f]chinolin-3(4H)-on als weisse Kristalle erhält, Smp. 180-181°.

b) 113,3 g (0,465 Mol) rac-1,2,5,6-Tetrahydro-2-methyl-7-methoxy[f]chinolin-3(4H)-on werden in 1,4 l Methylenchlorid gelöst und mit 222,5 ml (1,4 Mol) Triäthylsilan bei 20° versetzt. Anschliessend kühlt man auf 5° und tropft bei dieser Temperatur 555 ml Trifluoressigsäure zu. Die erhaltene klare Lösung wird 63 Stunden bei 20° gerührt, anschliessend auf 0,5 l gesättigte Natriumbicarbonatlösung gegossen und mit 28%iger Natronlauge auf pH 9 gestellt. Extraktion mit Methylenchlorid liefert 114,9 g eines kristallinen Rückstandes, der aus Methylenchlorid/Aethanol/Methanol umkristallisiert wird, wobei 101,1 g (89%) rac-1,2,4aα,5,6,10bβ-Hexahydro-2α-methyl -7-methoxybenzo[f]chinolin-3(4H)-on als weisse Kristalle erhalten werden, Smp. 248-250°.

c) 55,4 g (0,226 Mol) rac-1,2,4aα,5,6,10bβ-Hexahydro-2α-methyl -7-methoxybenzo[f]chinolin-3(4H)-on werden portionenweise zu einer Suspension von 17,2 g (0,452 Mol) Lithiumaluminiumchlorid in 2 l Tetrahydrofuran gegeben und anschliessend 1 Stunde unter Rückfluss gerührt. Nach Abkühlen auf 5° wird mit 250 ml gesättigter Ammoniumchloridlösung hydrolysiert, dann werden 500 ml Essigester hinzugefügt und über Kieselgur filtriert. Das Filtrat wird zwischen Essigester und Wasser verteilt, und die organische

Phase über Magnesiumsulfat getrocknet und unter vermindertem Druck eingedampft. Der Rückstand (53,5 g) wird in 300 ml Diäthyläther aufgekocht, filtriert, und das Filtrat mit 225 ml n-Hexan verdünnt. Nach Einengen dieser Lösung auf 175 ml erfolgt Kristallisation im Kühlschrank bei 4°. Man erhält auf diese Weise 28,5 g (54%) rac-1,2,3,4,4aα,5,6,10b β-Octahydro-2α-methyl-7-methoxybenzo[f]chinolin als weisse Kristalle, Smp. 81-83°.

d) Zu einer Lösung von 52 g (22,6 mMol) rac-1,2,3,4,4aα,5,6,10bβ-Octahydro-2α-methyl -7-methoxybenzo[f]chinolin in 80 ml Methylenchlorid werden zunächst 4,6 ml Triäthylamin und danach 2,3 ml (25,9 mMol) Propionsäurechlorid in 15 ml Methylenchlorid gegeben. Das Reaktionsgemisch wird danach 5 Stunden bei 20° in einer Argonatmosphäre gerührt. Anschliessend verteilt man zwischen gesättigter Natriumbicarbonatlösung und Methylenchlorid, trocknet die organische Phase mit wasserfreiem Magnesiumsulfat und destilliert die flüchtigen Anteile unter vermindertem Druck ab. Der Rückstand wird mit Methylenchlorid, welches 2% Methanol ent hält. als Eluierungsmittels an Kieselgel chromatographiert. Die Hauptkomponente (6,8 g) wird in 60 ml trockenem Tetrahydrofuran gelöst und innerhalb von 30 Minuten zu einer Suspension von 1,8 g Lithiumaluminiumhydrid in 100 ml Tetrahydrofuran bei 20-34° getropft. Anschliessend erhitzt man 1 Stunde zum Rückfluss, kühlt dann ab und hydrolysiert mit 20 ml gesättigter Ammoniumchloridlösung. Nach Zugabe von 50 ml Essigester wird 15 Minuten bei 20° weitergerührt und schliesslich über Kieselgur filtriert. Das Filtrat verteilt man zwischen Essigester und Wasser, trocknet die organische Phase über wasserfreiem Magnesiumsulfat und destilliert die flüchtigen Anteile unter vermindertem Druck ab. Man erhält auf diese Weise 6,0 g (98%) rac-1,2,3,4,4aα,5,6,10bβ-Octahydro-2α-methyl-4-propyl -7-methoxybenzo[f]chinolin als gelbes Oel, welches direkt in die nächste Stufe eingesetzt wird.

## Beispiel 2

3,9 g (13 mMol) rac-1,2,3,4,4aα,5,6,10bβ-Octahydro-2α-methyl -4-(2,2-dimethylpropyl)-7-methoxybenzo-[f]chinolin werden in 150 ml Bromwasserstoffsäure (48%) unter Rühren in einer Argonatmosphäre 1 Stunde zum Rückfluss erhitzt. Nach dem Abkühlen destilliert man die überschüssige Bromwasserstoffsäure unter vermindertem Druck ab und verteilt den Rückstand zwischen Methylenchlorid und gesättigter wässriger Natriumbicarbonatlösung. Die wässrige Phase wird mit 2N Natronlauge auf pH 9 gestellt, und die organische Phase abgetrennt. Nach zweimaliger Extraktion der wässrigen Phase mit Methylenchlorid werden die organischen Extrakte vereinigt, über wasserfreiem Magnesiumsulfat getrocknet und unter vermindertem Druck eingedampft. Der Rückstand wird in 150 ml Methanol gelöst, filtriert und mit Chlorwasserstoff in Aethanol angesäuert. Beim Einengen der Lösung auf 75 ml Volumen kristallisieren 3,7 g (87%) rac-1,2,3,4,4aα,5,6,10bβ-Octahydro-2α-methyl -4-(2,2-dimethylpropyl)benzo[f] chinolin-7-ol-hydrochlorid aus, Smp. 268-271°.

Das als Ausgangsmaterial verwendete rac-1,2,3,4,4aα,5,6,10bβ-Octahydro-2α-methyl -4-(2,2-dimethylpropyl)-7-methoxybenzo[f]chinolin wurde in analoger Weise wie in Beispiel 1d) beschrieben durch Umsetzen von rac-1,2,3,4,4aα,5,6,10bβ-Octahydro-2α-methyl -7-methoxybenzo[f]chinolin mit Pivalinsäurechlorid und anschliessender Reduktion mit Lithiumaluminiumhydrid erhalten und direkt in die nächste Stufe eingesetzt.

## Beispiel 3

3,6 g (12,5 mMol) rac-1,2,3,4,4aα,5,6,10bβ-Octahydro-2α-methyl -4-butyl-7-methoxybenzo[f]chinolin werden in 150 ml Bromwasserstoffsäure (48%) unter Rühren in einer Argonatmosphäre 1 Stunde zum Rückfluss erhitzt. Nach dem Abkühlen destilliert man die überschüssige Bromwasserstoffsäure unter vermindertem Druck ab und verteilt den Rückstand zwischen Methylenchlorid und gesättigter wässriger Natriumbicarbonatlösung. Die wässrige Phase wird mit 2N Natronlauge auf pH 9 gestellt, und die organische Phase abgetrennt. Nach zweimaliger Extraktion der wässrigen Phase mit Methylenchlorid werden die organischen Extrakte vereinigt, über wasserfreiem Magnesiumsulfat getrocknet und unter vermindertem Druck eingedampft. Der Rückstand wird in 150 ml Methanol gelöst, filtriert und mit Chlorwasserstoff in Aethanol angesäuert. Beim Einengen der Lösung auf 75 ml Volumen kristallisieren 3,5 g (90%) rac-1,2,3,4,4aα,5,6,10bβ-Octahydro-2α-methyl -4-butylbenzo[f]chinolin-7-ol-hydrochlorid aus, Smp. 250-254°.

Das als Ausgangsmaterial verwendete rac-1,2,3,4,4aα,5,6,10bβ-Octahydro-2α-methyl -4-butyl-7-methoxybenzo[f]chinolin wurde in analoger Weise wie in Beispiel 1d) beschrieben durch Umsetzen von rac-1,2,3,4,4aα,5,6,10bβ-Octahydro-2α-methyl -7-methoxy benzo[f]chinolin mit Buttersäurechlorid und anschliessender Reduktion mit Lithiumaluminiumhydrid erhalten und direkt in die nächste Stufe eingesetzt.

## Beispiel 4

3,2 g (13 mMol) rac-1,2,3,4,4aα,5,6,10bβ-Octahydro-2α-methyl -4-methyl-7-methoxybenzo[f]chinolin werden in 150 ml Bromwasserstoffsäure (48%) unter Rühren in einer Argonatmosphäre l 1/2 Stunden zum Rückfluss erhitzt. Nach dem Abkühlen destilliert man die überschüssige Bromwasserstoffsäure unter vermindertem Druck ab und verteilt den Rückstand zwischen Methylenchlorid und gesättigter wässriger Natriumbicarbonatlösung. Die wässrige Phase wird mit 2N Natronlauge auf pH 9 gestellt, und die organische Phase abgetrennt. Nach zweimaliger Extraktion der wässrigen Phase mit Methylenchlorid werden die organischen Extrakte vereinigt, über wasserfreiem Magnesiumsulfat getrocknet und unter vermindertem Druck eingedampft. Der Rückstand wird in 150 ml Methanol gelöst, filtriert und mit Chlorwasserstoff in Aethanol angesäuert. Beim Einengen der Lösung auf 50 ml Volumen kristallisieren 2,9 g (83%) rac-1,2,3,4,4aα,5,6,10bβ-Octahydro-2α-methyl -4-methylbenzo[f]chinolin-7-ol-hydrochlorid aus, Smp. 275-278°.

Das als Ausgangsmaterial verwendete rac-1,2,3,4,4aα,5,6,10bβ-Octahydro-2α-methyl -4-methyl-7-methoxybenzo[f]chinolin wurde aus rac-1,2,3,4,4aα,5,6,10bβ-Octahydro-2α-methyl -7-methoxybenzo[f]-chinolin durch reduktive Methylierung mit einem 1:2-Gemisch von Formaldehyd und Ameisensäure bei 100° (2 Stunden) hergestellt und direkt in die nächste Stufe eingesetzt.

## Beispiel 5

1,9 g (6,5 mMol) rac-1,2,3,4,4aα,5,6,10bβ-Octahydro-2α-methyl -4-(3-hydroxypropyl)-7-methoxybenzo[f]-chinolin werden in 100 ml Bromwasserstoffsäure (48%) unter Rühren in einer Argonatmosphäre 2 Stunden zum Rückfluss erhitzt. Nach dem Abkühlen destilliert man die überschüssige Bromwasserstoffsäure unter vermindertem Druck ab. Der Rückstand wird in 50 ml Methanol gelöst, filtriert und mit 50 ml Essigester versetzt. Beim Einengen der Lösung auf 25 ml Volumen kristallisieren 1,65 g (61%) rac-1,2,3,4,4aα,5,6,10bβ-Octahydro-2α-methyl -4-(3-brompropyl)benzo[f]chinolin-7-ol-hydrobromid aus, Smp. 248-251°.

Das als Ausgangsmaterial verwendete rac-1,2,3,4,4aα,5,6,10bβ-Octahydro-2α-methyl-4-(3-hydroxypropyl)-7-methoxybenzo[f]chinolin wurde in analoger Weise wie in Beispiel 30 beschrieben durch Umsetzen von rac-1,2,3,4,4aα,5,6,10bβ-Octahydro-2α-methyl-7-methoxybenzo[f]chinolin mit Acrylsäuremethylester und anschliessender Reduktion mit Lithiumaluminiumhydrid, wie in Beispiel 33 beschrieben, hergestellt und direkt in die nächste Stufe eingesetzt.

## Beispiel 6

2,7 g (6,8 mMol) rac-[1,2,3,4,4aα,5,6,10bβ-Octahydro-2α-methyl -7-methoxybenzo[f]chinolin-4-yl]-4′-fluor-butyrophenon werden in 400 ml Bromwasserstoffsäure (48%) unter Rühren in einer Argonatmosphäre 5 Stunden zum Rückfluss erhitzt. Nach dem Abkühlen destilliert man die überschüssige Bromwasserstoffsäure unter vermindertem Druck ab und verteilt den Rückstand zwischen Methylenchlorid und gesättigter wässriger Natriumbicarbonatlösung. Die wässrige Phase wird mit 2N Natronlauge auf pH 9 gestellt, und die organische Phase abgetrennt. Nach zweimaliger Extraktion der wässrigen Phase mit Methylenchlorid werden die organischen Extrakte vereinigt, über wasserfreiem Magnesiumsulfat getrocknet und unter vermindertem Druck eingedampft. Der Rückstand wird in 10 ml Aethanol gelöst, filtriert und mit Chlorwasserstoff in Aethanol angesäuert. Bei Zugabe von 30 ml Essigester kristallisieren 2,05 g (62%) rac-[1,2,3,4,4aα,5,6,10bβ-Octahydro-2α-methyl -7-hydroxybenzo[f]chinolin-4-yl]-4′-fluorbutyrophenon - hydrochlorid aus, Smp. 170-172°.

Das als Ausgangsmaterial verwendete rac-[1,2,3,4,4aα,5,6,10bβ-Octahydro-2α-methyl -7-methoxybenzo-[f]chinolin-4-yl]-4′-fluor-butyrophenon wurde in analoger Weise wie in Beispiel 31 beschrieben durch

Umsetzen von rac-1,2,3,4,4aα,5,6,10bβ-Octahydro-2α-methyl -7-methoxybenzo[f]chinolin und 4-Chlor-4'-fluorbutyrophenon hergestellt und direkt in die nächste Stufe eingesetzt.

## Beispiel 7

7,1 g (19,1 mMol) rac-1,2,3,4,4aα,5,6,10bβ-Octahydro-2α-methyl -4-(4-chlorphenäthyl)-7-methoxybenzo[f]chinolin werden in 400 ml Bromwasserstoffsäure (48%) unter Rühren in einer Argonatmosphäre 1 Stunde zum Rückfluss erhitzt. Nach dem Abkühlen destilliert man die überschüssige Bromwasserstoffsäure unter vermindertem Druck ab und verteilt den Rückstand zwischen Methylenchlorid und gesättigter wässriger Natriumbicarbonatlösung. Die wässrige Phase wird mit 2N Natronlauge auf pH 9 gestellt, und die organische Phase abgetrennt. Nach zweimaliger Extraktion der wässrigen Phase mit Methylenchlorid werden die organischen Extrakte vereinigt, über wasserfreiem Magnesiumsulfat getrocknet und unter vermindertem Druck eingedampft. Der Rückstand wird in 200 ml Aethanol gelöst, filtriert und mit Chlorwasserstoff in Aethanol angesäuert. Beim Einengen der Lösung auf 50 ml Volumen kristallisieren 5,9 g (79%) rac-1,2,3,4,4aα,5,6,10bβ-Octahydro-2α-methyl -4-(4-chlorphenäthyl)benzo[f]chinolin-7-ol-hydrochlorid aus, Smp. 264-266° C.

Das als Ausgangsmaterial verwendete rac-1,2,3,4,4aα,5,6,10bβ-Octahydro-2α-methyl -4-(4-chlorphenäthyl)-7-methoxybenzo[f]chinolin wurde aus rac-1,2,3,4,4aα,5,6,10bβ-Octahydro-2α-methyl -7-methoxy benzo[f]chinolin und 4-Chlorphenylessigsäure durch Acylierung (vgl. Synthesis 1983, S. 1013) unter Verwendung von Boran-Trimethylamin in Xylol und nachfolgender Reduktion des intermediär gebildeten Amids mit Lithiumaluminiumhydrid erhalten und direkt in die nächste Stufe eingesetzt.

## Beispiel 8

3,6 g (10,6 mMol) rac-1,2,3,4,4aα,5,6,10bβ-Octahydro-2α-methyl -4-[2-(2-thienyl)äthyl]-7-methoxybenzo[f]chinolin werden in 400 ml Bromwasserstoffsäure (48%) unter Rühren in einer Argonatmosphäre 2 Stunden zum Rückfluss erhitzt. Nach dem Abkühlen destilliert man die überschüssige Bromwasserstoffsäure unter vermindertem Druck ab und verteilt den Rückstand zwischen Methylenchlorid und gesättigter wässriger Natriumbicarbonatlösung. Die wässrige Phase wird mit 2N Natronlauge auf pH 9 gestellt, und die organische Phase abgetrennt. Nach zweimaliger Extraktion der wässrigen Phase mit Methylenchlorid werden die organischen Extrakte vereinigt, über wasserfreiem Magnesiumsulfat getrocknet und unter vermindertem Druck eingedampft. Der Rückstand wird in 200 ml Methanol gelöst, filtriert und mit Chlorwasserstoff in Aethanol angesäuert. Bei Zugabe von 50 ml Essigester und Einengen auf 40 ml Volumen kristallisieren 1,95 g (51%) rac-1,2,3,4,4aα,5,6,10bβ-Octahydro-2α-methyl -4-[2-(2-thienyl)äthyl]-benzo[f]chinolin-7-ol-hydrochlorid aus, Smp. 276-279°.

Das als Ausgangsmaterial verwendete rac-1,2,3,4,4aα-5,6,10bβ-Octahydro-2α-methyl -4-[2-(2-thienyl)-äthyl]-7-methoxybenzo[f]chinolin wurde aus rac-1,2,3,4,4aα,5,6,10bβ-Octahydro-2α-methyl -7-methoxybenzo[f]chinolin und Thiophen-2-essigsäure durch Acylierung (vgl. Synthesis 1983, S. 1013) unter Verwendung von Boran-Trimethylamin in Xylol und anschliessender Reduktion des intermediär gebildeten Amids mit Lithiumaluminiumhydrid er halten und direkt in die nächste Stufe eingesetzt.

## Beispiel 9

4,2 g (12 mMol) rac-1,2,3,4,4aα,5,6,10bβ-Octahydro-2α-methyl -4-(4-methylphenäthyl)-7-methoxybenzo[f]chinolin werden in 250 ml Bromwasserstoffsäure (48%) unter Rühren in einer Argonatmosphäre 4 Stunden zum Rückfluss erhitzt. Nach dem Abkühlen destilliert man die überschüssige Bromwasserstoffsäure unter vermindertem Druck ab und verteilt den Rückstand zwischen Methylenchlorid und gesättigter wässriger Natriumbicarbonatlösung. Die wässrige Phase wird mit 2N Natronlauge auf pH 9 gestellt, und die organische Phase abgetrennt. Nach zweimaliger Extraktion der wässrigen Phase mit Methylenchlorid werden die organischen Extrakte vereinigt, über wasserfreiem Magnesiumsulfat getrocknet und unter vermindertem Druck eingedampft. Der Rückstand wird in 100 ml Aethanol gelöst, filtriert und mit Chlorwasserstoff in Aethanol angesäuert. Beim Einengen der Lösung auf 50 ml Volumen kristallisieren 3,4 g (76%)

rac-1,2,3,4,4aα,5,6,10bβ-Octahydro-2α-methyl -4-(4-methylphenäthyl]benzo[f]chinolin-7-ol-hydrochlorid aus, Smp. 285-288°.

Das als Ausgangsmaterial verwendete rac-1,2,3,4,4aα,5,6,10bβ-Octahydro-2α-methyl -4-(4-methylphenäthyl)-7-methoxybenzo[f]chinolin wurde aus rac-1,2,3,4,4aα,5,6,10bβ-Octahydro-2α-methyl -7-methoxybenzo[f]chinolin und p-Tolylessigsäure durch Acylierung (vgl. Synthesis 1983, S. 1013) unter Verwendung von Boran-Trimethylamin in Xylol und anschliessender Reduktion des intermediär gebildeten Amids mit Lithiumaluminiumhydrid erhalten und direkt in die nächste Stufe eingesetzt.

## Beispiel 10

4,25 g (11,6 mMol) rac-1,2,3,4,4aα,5,6,10bβ-Octahydro-2α-methyl -4-(4-methoxyphenäthyl)-7-methoxybenzo[f] chinolin werden in 250 ml Bromwasserstoffsäure (48%) unter Rühren in einer Argonatmosphäre 1 1/2 Stunden zum Rückfluss erhitzt. Nach dem Abkühlen destilliert man die überschüssige Bromwasserstoffsäure unter vermindertem Druck ab und verteilt den Rückstand zwischen Methylenchlorid und gesättigter wässriger Natriumbicarbonatlösung. Die wässrige Phase wird mit 2N Natronlauge auf pH 9 gestellt, und die organische Phase abgetrennt. Nach zweimaliger Extraktion der wässrigen Phase mit Methylenchlorid werden die organischen Extrakte vereinigt, über wasserfreiem Magnesiumsulfat getrocknet und unter vermindertem Druck eingedampft. Der Rückstand wird in 100 ml Aethanol gelöst, filtriert und mit Chlorwasserstoff in Aethanol angesäuert. Beim Einengen der Lösung auf 50 ml Volumen kristallisieren 3,3 g (76%) rac-1,2,3,4,4aα,5,6,10bβ-Octahydro-2α-methyl -4-(4-hydroxyphenäthyl)benzo[f]chinolin-7-ol-hydrochlorid aus, Smp. 304-308°.

Das als Ausgangsmaterial verwendete rac-1,2,3,4,4aα,5,6,10bβ-Octahydro-2α-methyl -4-(4-methoxyphenäthyl)-7-methoxybenzo[f]chinolin wurde aus rac-1,2,3,4,4aα,5,6,10bβ-Octahydro-2α-methyl -7-methoxybenzo[f]chinolin und p-Methoxyphenylessigsäure durch Acylierung (vgl. Synthesis 1983, S. 1013) unter Verwendung von Boran-Trimethylamin in Xylol und anschliessender Reduktion des intermediär gebildeten Amids mit Lithiumaluminiumhydrid erhalten und direkt in die nächste Stufe eingesetzt.

## Beispiel 11

3,55 g (12 mMol) rac-1,2,3,4,4aα,5,6,10bβ-Octahydro-2α-methyl -4-(2-methoxyäthyl)-7-methoxybenzo[f]-chinolin werden in 140 ml Bromwasserstoffsäure (48%) unter Rühren in einer Argonatmosphäre 1/2 Stunde zum Rückfluss erhitzt. Nach dem Abkühlen destilliert man die überschüssige Bromwasserstoffsäure unter vermindertem Druck ab und verteilt den Rückstand zwischen Methylenchlorid und gesättigter wässriger Natriumbicarbonatlösung. Die wässrige Phase wird mit 2N Natronlauge auf pH 9 gestellt, und die organische Phase abgetrennt. Nach zweimaliger Extraktion der wässrigen Phase mit Methylenchlorid werden die organischen Extrakte vereinigt, über wasserfreiem Magnesiumsulfat getrocknet und unter vermindertem Druck eingedampft. Der Rückstand wird in 50 ml Methanol gelöst, filtriert und mit Chlorwasserstoff in Aethanol angesäuert. Bei Zugabe von 2 ml Chlorwasserstoff in Aethanol (6N) kristallisieren 1,8 g (50%) rac-1,2,3,4,4aα,5,6,10bβ-Octahydro-2α-methyl -7-hydroxybenzo[f]chinolin-4-äthanol-hydrochlorid aus, Smp. 252-253°.

Das als Ausgangsmaterial verwendete rac-1,2,3,4,4aα,5,6,10bβ-Octahydro-2α-methyl -4-(2-methoxyäthyl)-7-methoxybenzo[f]chinolin wurde in analoger Weise wie in Beispiel 1d) beschrieben durch Umsetzen von rac-1,2,3,4,4aα,5,6,10bβ-Octahydro-2α-methyl -7-methoxybenzo[f]chinolin mit Methoxyessigsäurechlorid und anschliessender Reduktion mit Lithiumaluminiumhydrid erhalten und direkt in die nächste Stufe eingesetzt.

## Beispiel 12

1,45 g (3,6 mMol) rac-1,2,3,4,4aα,5,6,10bβ-Octahydro-2α-methyl -4-(4-indol-3-ylbutyl)-7-methoxybenzo-[f]chinolin werden in 35 ml Methylenchlorid unter einer Argonatmosphäre mit 1,1 ml Bortribromid 1 Stunde bei -10° und 1 Stunde bei 10-15° gerührt. Danach werden 30 ml 2N Natronlauge zugetropft und 15 Minuten bei 20° weitergerührt. Die wässrige Phase wird dann mit gesättigter Ammoniumchloridlösung auf

pH 8 gestellt, und die organische Phase abgetrennt. Nach zweimaliger Extraktion der wässrigen Phase mit Methylenchlorid werden die organischen Extrakte vereinigt, über wasserfreiem Magnesiumsulfat getrocknet und unter vermindertem Druck eingedampft. Der Rückstand wird in 10 ml Aethanol gelöst, filtriert und mit Chlorwasserstoff in Aethanol angesäuert. Bei Zugabe von 2 ml Chlorwasserstoff in Aethanol (6N) kristallisieren 0,85 g (55%) rac-1,2,3,4,4aα,5,6,10bβ-Octahydro-2α-methyl -4-(4- indol-3-ylbutyl)benzo[f]chinolin7-ol-hydrochlorid aus, Smp. 158-163°.

Das als Ausgangsmaterial verwendete rac-1,2,3,4,4aα,5,6,10bβ-Octahydro-2α-methyl -4-(4-indol-3-ylbutyl)-7-methoxybenzo[f]chinolin wurde aus rac-1,2,3,4,4aα,5,6,10bβ-Octahydro-2α-methyl -7-methoxybenzo-[f]chinolin und 4-(3-Indolyl)buttersäure durch Acylierung (vgl. Synthesis 1983, S. 1013) unter Verwendung von Boran-Trimethylamin in Xylol und anschliessender Reduktion des intermediär gebildeten Amids mit Lithiumaluminiumhydrid erhalten und direkt in die nächste Stufe eingesetzt.

## Beispiel 13

Zu einer Lösung von 0,9 g (3,3 mMol) rac-1,2,3,4,4aα,5,6,10bβ-Octahydro-2α-methyl-4-allyl-7-methoxybenzo[f]chinolin in 15 ml Methylenchlorid gibt man bei -78° 1,05 ml Bortribromid in 10 ml Methylenchlorid und rührt das Gemisch 2 Stunden bei 20°. Anschliessend werden bei 0° 16 ml 2N Natronlauge zugetropft, und wird 1/4 Stunde bei 20° weitergerührt. Die hydrolysierte Lösung wird mit Ammoniumchlorid auf pH 8-9 gestellt und mit Methylenchlorid extrahiert. Die vereinigten Extrakte trocknet man über Magnesiumsulfat und dampft ein. Chromatographie des Rückstandes an Kieselgel mit Methylenchlorid/Methanol (98:2) liefert 0,65 g rac-1,2,3,4,4aα,5,6,10bβ-Octahydro-2α-methyl-4-allyl -7-hydroxybenzo[f]chinolin, das mit Chlorwasserstoff ins Hydrochlorid übergeführt wird, Ausbeute 0,66 g (68%), Smp. 267-270°.

Das als Ausgangsmaterial verwendte rac-1,2,3,4,4aα,5,6,10bβ-Octahydro-2α-methyl-4-allyl-7-methoxybenzo[f]chinolin wurde in analoger Weise wie in Beispiel 1d) beschrieben durch Umsetzen von rac-1,2,3,4,4aα,5,6,10bβ-Octahydro-2α-methyl -7-methoxybenzo[f]chinolin mit Acrylsäurechlorid und anschliessender Reduktion mit Lithiumaluminiumhydrid erhalten und direkt in die nächste Stufe eingesetzt.

## Beispiel 14

Zu einer Lösung von 3,9 g (13 mMol) rac-1,2,3,4,4aα,5,6,10bβ-Octahydro-2α-methyl -7-methoxybenzo-[f]chinolin-4-butyronitril in 40 ml Methylenchlorid gibt man bei -10° 3,75 ml Bortribromid in 30 ml Methylenchlorid und rührt das Gemisch 16 Stunden bei 20°. Anschliessend werden bei 0° 80 ml 2N Natronlauge zugetropft, und wird 1/2 Stunde bei 20° weitergerührt. Die hydrolysierte Lösung wird mit Ammoniumchloridlösung auf pH 8-9 gestellt und mit Methylenchlorid extrahiert. Die vereinigten Extrakte trocknet man über Magnesiumsulfat und dampft ein. Chromatographie des Rückstandes an Kieselgel mit Methylenchlorid/Methanol (99:1) liefert 2,65 g rac-1,2,3,4,4aα,5,6,10bβ-Octahydro-2α-methyl -7-hydroxybenzo[f]chinolin-4-butyronitril, das mit Chlorwasserstoff ins Hydrochlorid übergeführt wird, Ausbeute 2,24 g (54%), Smp. 255-257°.

Das als Ausgangsmaterial verwendete rac-1,2,3,4,4aα,5,6,10bβ-Octahydro-2α-methyl -7-methoxybenzo-[f]chinolin-4-butyronitril wurde in analoger Weise wie in Beispiel 31 beschrieben durch Umsetzen von rac-1,2,3,4,4aα,5,6,10bβ-Octahydro-2α-methyl -7-methoxybenzo[f]chinolin mit 4-Brombutyronitril hergestellt und direkt in die nächste Stufe eingesetzt.

## Beispiel 15

3 g (13 mMol) rac-1,2,3,4,4aα,5,6,10bβ-Octahydro-2α-methyl -7-methoxybenzo[f]chinolin in 100 ml Aethylmethylketon werden mit 5,6 ml (52 mMol) Cyclopentylbromid, 3,6 g Kaliumcarbonat und 0,9 g Natriumjodid 113 Stunden zum Rückfluss erhitzt. Anschliessend destilliert man die flüchtigen Anteile unter vermindertem Druck ab und verteilt den Rück stand zwischen gesättigter Natriumbicarbonatlösung und Essigester. Der Rückstand aus der organischen Phase wird an Kieselgel chromatographiert (Methylenchlorid/Methanol 98:2, V/V).

Die Hauptkomponente aus der Chromatographie (3,15 g) löst man in 120 ml Bromwasserstoffsäure (48%) und erhitzt 1 Stunde zum Rückfluss. Nach dem Abkühlen werden die flüchtigen Anteile unter vermindertem Druck entfernt, und der Rückstand zwischen gesättigter Natriumbicarbonatlösung und Methylenchlorid verteilt. Nach dem Trocknen der organischen Phase über Magnesiumsulfat und Eindampfen werden 2,7 g der kristallinen Base erhalten, Smp. 199-202°. Diese werden mit Chlorwasserstoff in Aethanol ins Hydrochlorid übergeführt, wobei man 2,5 g (65%) rac-1,2,3,4,4aα,5,6,10bβ-Octahydro-2α-methyl -4-cyclopentylbenzo[f]chinolin-7-ol-hydrochlorid erhält, Smp. >310°.

## Beispiel 16

3,0 g (13 mMol) rac-1,2,3,4,4aα,5,6,10bβ-Octahydro-2α-methyl -7-methoxybenzo[f]chinolin in 100 ml Aethylmethylketon werden mit 5,0 ml (52 mMol) Isopropylbromid, 3,6 g Kaliumcarbonat und 0,9 g Natriumjodid 113 Stunden zum Rückfluss erhitzt. Anschliessend destilliert man die flüchtigen Anteile unter vermindertem Druck ab und verteilt den Rückstand zwischen gesättigter Natriumbicarbonatlösung und Essigester. Der Rückstand aus der organischen Phase wird an Kieselgel chromatographiert (Methylenchlorid/Methanol 97:3).

Die Hauptkomponente aus der Chromatographie (2,8 g) löst man in 170 ml Bromwasserstoffsäure (48%) und erhitzt 1 Stunde zum Rückfluss. Nach dem Abkühlen werden die flüchtigen Anteile unter vermindertem Druck entfernt, und der Rückstand zwischen gesättigter Natriumbicarbonatlösung und Methylenchlorid verteilt. Nach dem Trocknen der organischen Phase über Magnesiumsulfat und Eindampfen werden 2,6 g der kristallinen Base erhalten, welche mit Chlorwasserstoff in Aethanol ins Hydrochlorid übergeführt werden, wobei man 2,94 g (66%) rac-1,2,3,4,4aα,5,6,10bβ-Octahydro-2α-methyl -4-isopropylbenzo[f]chinolin-7-ol-hydrochlorid erhält, Smp. >305°.

## Beispiel 17

3,0 g (13 mMol) rac-1,2,3,4,4aα,5,6,10bβ-Octahydro-2α-methyl -7-methoxybenzo[f]chinolin in 100 ml Aethylmethylketon werden mit 2,8 ml (26 mMol) 3-Cyclohexylpropylchlorid, 3,6 g Kaliumcarbonat und 0,9 g Natriumjodid 20 Stunden zum Rückfluss erhitzt. Anschliessend destilliert man die flüchtigen Anteile unter vermindertem Druck ab und verteilt den Rückstand zwischen gesättigter Natriumbicarbonatlösung und Essigester. Der Rückstand aus der organischen Phase wird an Kieselgel chromatographiert (Methylenchlorid/Methanol 99:1).

Die Hauptkomponente aus der Chromatographie (4,7 g) löst man in 180 ml Bromwasserstoffsäure (48%) und erhitzt 1 Stunde zum Rückfluss. Nach dem Abkühlen werden die flüchtigen Anteile unter vermindertem Druck entfernt. Der Rückstand wird in je 150 ml heissem Methanol und Aethanol gelöst, mit Aktivkohle aufgekocht und über Kieselgur filtriert. Nach Einengen unter vermindertem Druck auf 70 ml Volumen kristallisieren 3,2 g (58%) rac-1,2,3,4,4aα,5,6,10bβ-Octahydro-2α-methyl-4-(3-cyclohexylpropyl)-benzo[f]chinolin-7-ol-hydrobromid aus, Smp. 256-257°.

## Beispiel 18

2,3 g (10 mMol) rac-1,2,3,4,4aα,5,6,10bβ-Octahydro-2α-methyl -7-methoxybenzo[f]chinolin in 100 ml Aethylmethylketon werden mit 4,0 g (20 mMol) 3-Phenylpropylbromid, 3,6 g Kaliumcarbonat und 0,9 g Natriumjodid 20 Stunden zum Rückfluss erhitzt. Anschliessend destilliert man die flüchtigen Anteile unter vermindertem Druck ab und verteilt den Rückstand zwischen gesättigter Natriumbicarbonatlösung und Essigester. Der Rückstand aus der organischen Phase wird an Kieselgel chromatographiert (Methylenchlorid/Methanol 99:1).

Die Hauptkomponente aus der Chromatographie (4,35 g) löst man in 200 ml Bromwasserstoffsäure (48%) und erhitzt 1 Stunde zum Rückfluss. Nach dem Abkühlen werden die flüchtigen Anteile unter vermindertem Druck entfernt, und der Rückstand zwischen gesättigter Natriumbicarbonatlösung und Methylenchlorid verteilt. Nach dem Trocknen der organischen Phase über Magnesiumsulfat und Einengen werden 3,15 g der kristallinen Base erhalten, welche mit Chlorwasserstoff in Aethanol ins Hydrochlorid übergeführt

werden, wobei man 3,19 g (86%) rac-1,2,3,4,4aα,5,6,10bβ-Octahydro-2α-methyl -4-(3-phenylpropyl)benzo[f]-chinolin-7-ol-hydrochlorid erhält, Smp. 264-270°.

Beispiel 19

3,0 g (13 mMol) rac-1,2,3,4,4aα,5,6,10bβ-Octahydro-2α-methyl -7-methoxybenzo[f]chinolin in 100 ml Aethylmethylketon werden mit 5 g (26 mMol) 2-Cyclohexyläthylbromid, 3,6 g Kaliumcarbonat und 0,9 g Natriumjodid 20 Stunden zum Rückfluss erhitzt. Anschliessend destilliert man die flüchtigen Anteile unter vermindertem Druck ab und verteilt den Rückstand zwischen gesättigter Natriumbicarbonatlösung und Essigester. Der Rückstand aus der organischen Phase wird an Kieselgel chromatographiert (Methylenchlorid/Methanol 98:2).

Die Hauptkomponente aus der Chromatographie (3,65 g) löst man in 200 ml Bromwasserstoffsäure (48%) und erhitzt 1 Stunde zum Rückfluss. Nach dem Abkühlen werden die flüchtigen Anteile unter vermindertem Druck entfernt, und der Rückstand zwischen gesättigter Natriumbicarbonatlösung und Methylenchlorid verteilt. Nach dem Trocknen der organischen Phase über Magnesiumsulfat und Eindampfen werden 3,4 g der Base erhalten, welche mit Chlorwasserstoff in Aethanol ins Hydrochlorid übergeführt werden, wobei man 2,9 g (57%) rac- 1,2,3,4,4aα,5,6,10bβ-Octahydro-2α-methyl -4-(2-cyclohexyläthyl)benzo-[f]chinolin-7-ol-hydrochlorid erhält, Smp. 278-280°.

Beispiel 20

3,0 g (13 mMol) rac-1,2,3,4,4aα,5,6,10bβ-Octahydro-2α-methyl -7-methoxybenzo[f]chinolin in 100 ml Aethylmethylketon werden mit 6,53 g (26 mMol) 4-Chlor-1-(4-t-butylphenyl)-1-butanon (95%), 3,6 g Kaliumcarbonat und 0,9 g Natriumjodid 20 Stunden zum Rückfluss erhitzt. Anschliessend destilliert man die flüchtigen Anteile unter vermindertem Druck ab und verteilt den Rückstand zwischen gesättigter Natriumbicarbonatlösung und Essigester. Der Rückstand aus der organischen Phase wird an Kieselgel chromatographiert (Essigester/n-Hexan 1:1).

Die Hauptkomponente aus der Chromatographie (4,2 g) löst man in 300 ml Bromwasserstoffsäure (48%) und erhitzt 3 1 2 Stunden zum Rückfluss. Nach dem Abkühlen werden die flüchtigen Anteile unter vermindertem Druck entfernt, und der Rückstand zwischen gesättigter Natriumbicarbonatlösung und Methylenchlorid verteilt. Nach dem Trocknen der organischen Phase über Magnesiumsulfat und Eindampfen werden 3,95 g der Base erhalten, welche in 100 ml Essigester mit Chlorwasserstoff in Aethanol ins Hydrochlorid übergeführt werden, wobei man 2,75 g (46%) rac-1,2,3,4,4aα,5,6,10bβ-Octahydro-2α-methyl -4-(4′-t-butyl-4-phenyl-4-oxobutyl)benzo[f]chinolin-7-ol-hydrochlorid erhält, Smp. 170-172°.

Beispiel 21

3,0 g (13 mMol) rac-1,2,3,4,4aα,5,6,10bβ-Octahydro-2α-methyl -7-methoxybenzo[f]chinolin in 100 ml Dimethylformamid werden mit 6,3 ml (52 mMol) Cyclohexylbromid, 3,6 g Kaliumcarbonat und 0,9 g Natriumjodid 120 Stunden zum Rückfluss erhitzt. Anschliessend destilliert man die flüchtigen Anteile unter vermindertem Druck ab und verteilt den Rückstand zwischen gesättigter Natriumbicarbonatlösung und Essigester. Der Rückstand aus der organischen Phase wird an Kieselgel chromatographiert (zuerst Essigester/n-Hexan 1:3, dann Essigester).

Die Hauptkomponente aus der Chromatographie (1,5 g) löst man in 100 ml Bromwasserstoffsäure (48%) und erhitzt 2 Stunden zum Rückfluss. Nach dem Abkühlen werden die flüchtigen Anteile unter vermindertem Druck entfernt, und der Rückstand zwischen gesättigter Natriumbicarbonatlösung und Methylenchlorid verteilt. Nach dem Trocknen der organischen Phase über Magnesiumsulfat und Eindampfen werden 1,3 g der Base erhalten, welche mit Chlorwasserstoff in Aethanol ins Hydrochlorid übergeführt werden, wobei man 0,95 g (22%) rac-1,2,3,4,4aα,5,6,10bβ-Octahydro-2α-methyl -4-cyclohexylbenzo[f]-chinolin-7-ol-hydrochlorid erhält, Smp. >305°.

Beispiel 22

3,0 g (13 mMol) rac-1,2,3,4,4aα,5,6,10bβ-Octahydro-2α-methyl -7-methoxybenzo[f]chinolin in 100 ml Aethylmethylketon werden mit 5,0 g (37 mMol) Cyclobutylbromid, 3,6 g Kaliumcarbonat und 0,9 g Natriumjodid 5 Tage im Autoklaven auf 150° erhitzt. Anschliessend destilliert man die flüchtigen Anteile unter vermindertem Druck ab und verteilt den Rückstand zwischen gesättigter Natriumbicarbonatlösung und Essigester.

Das Rohprodukt löst man in 300 ml Bromwasserstoffsäure (48%) und erhitzt 5 Stunden zum Rückfluss. Nach dem Abkühlen werden die flüchtigen Anteile unter vermindertem Druck entfernt, und der Rückstand zwischen gesättigter Natriumbicarbonatlösung und Methylenchlorid verteilt. Nach dem Trocknen der organischen Phase über Magnesiumsulfat und Eindampfen werden 3,35 g der Base erhalten, welche an Kieselgel chromatographiert werden (Methylenchlorid/Methanol 96:4 bis 92:8). Die Hauptkomponente (1,5 g) wird mit Chlorwasserstoff in Aethanol ins Hydrochlorid übergeführt, wobei man 1,05 g (23%) rac-1 ,2,3,4,4aα,5,6,10bβ-Octahydro-2α-methyl -4-cyclobutylbenzo[f]chinolin-7-ol-hydrochlorid erhält,. Smp. 280-283°.

Beispiel 23

3,0 g (13 mMol) rac-1,2,3,4,4aα,5,6,10bβ-Octahydro-2α-methyl -7-methoxybenzo[f]chinolin in 100 ml Aethylmethylketon werden mit 5,1 g (26 mMol) 4-Chlor-1-(4-methylphenyl)-1-butanon, 3,6 g Kaliumcarbonat und 0,9 g Natriumjodid 72 Stunden im Autoklaven auf 150° erhitzt. Anschliessend destilliert man die flüchtigen Anteile unter vermindertem Druck ab und verteilt den Rückstand zwischen gesättigter Natriumbicarbonatlösung und Essigester. Der Rückstand aus der organischen Phase wird an Kieselgel chromatographiert (zuerst Essigester/n-Hexan 1:3, dann Essigester).

Die Hauptkomponente aus der Chromatographie (1,3 g) löst man in 150 ml Bromwasserstoffsäure (48%) und erhitzt 5 Stunden zum Rückfluss. Nach dem Abkühlen werden die flüchtigen Anteile unter vermindertem Druck entfernt, und der Rückstand zwischen gesättigter Natriumbicarbonatlösung und Methylenchlorid verteilt. Nach dem Trocknen der organischen Phase über Magnesiumsulfat und Eindampfen werden 1,2 g der Base erhalten, welche mit Chlorwasserstoff in Aethanol ins Hydrochlorid übergeführt werden, wobei man 0,73 g (15%) rac-1,2,3,4,4aα,5,6,10bβ-Octahydro-2α-methyl -4-(4-methylphenyl-4-oxobutyl)benzo[f]chinolin -7-ol-hydrochlorid erhält, Smp. 173-175°.

Beispiel 24

2,94 g (15,6 mMol) (-)-1,2,3,4,4aα,5,6,10bβ-Octahydro-2α-methyl -7-methoxybenzo[f]chinolin in 120 ml Aethylmethylketon werden mit 10,05 ml (93,3 mMol) Cyclopentylbro mid, 8,6 g Kaliumcarbonat und 1 g Natriumjodid 72 Stunden zum Rückfluss erhitzt. Anschliessend wird die abgekühlte Reaktionslösung auf 100 ml Wasser gegossen und mit zweimal 100 ml Essigester extrahiert. Die über Magnesiumsulfat getrocknete organische Phase wird unter vermindertem Druck eingedampft, und der Rückstand an Kieselgel mit Methylenchlorid/Methanol (98:2) als Eluierungsmittel chromatographiert.

Die Hauptkomponente aus der Chromatographie (3,0 g) wird in 110 ml Bromwasserstoffsäure 2 Stunden zum Rückfluss erhitzt. Nach dem Abkühlen destilliert man die flüchtigen Anteile unter vermindertem Druck ab. Der Rückstand wird in 70 ml Methanol und 300 ml Methylenchlorid suspendiert, und zur Suspension unter Rühren soviel 2N Natronlauge zugegeben, bis eine Lösung erhalten wird. Anschliessend stellt man den pH-Wert der Lösung mit gesättigter Ammoniumchloridlösung auf 8 ein und extrahiert mit Methylenchlorid. Die über Magnesiumsulfat getrocknete organische Phase wird eingedampft, der Rückstand in 100 ml Methanol und 100 ml Aethanol gelöst und mit alkoholischer Chlorwasserstofflösung angesäuert und auf ca. 30 ml Volumen eingeengt, wobei 2,4 g (49%) beige Kristalle von (-)-1,2,3,4,4aα,5,6,10bβ-Octahydro-2α-methyl-4-cyclopentylbenzo[f]chinolin-7-ol-hydrochlorid auskristallisieren, Smp. 284-289°; $[\alpha]_D$ = -38,0° (c = 1% in Methanol).

Das als Ausgangsmaterial verwendete (-)-1,2,3,4,4aα,5,6,10bβ-Octahydro-2α-methyl -7-methoxybenz[f]-chinolin wurde wie folgt hergestellt:

Zu einer Lösung von 24,8 g (106 mMol) rac-1,2,3,4,4aα,5,6,10bβ-Octahydro-2α-methyl -7-methoxybenzo[f]chinolin in 500 ml Methylenchlorid und 50 ml Wasser werden unter Rühren gleichzeitig aus zwei Tropftrichtern 21,9 g (118 mMol) R-(-)-α-Methoxyphenylessigsäurechlorid [hergestellt aus R-(-)-α-Methoxyphenylessigsäure und Oxalylchlorid durch 1-stündiges Erhitzen in Methylenchlorid zum Rückfluss] in 50 ml Methylenchlorid und 500 ml 5%ige Natronlauge getropft und anschliessend 15 Stunden bei 20° weitergerührt. Zur Aufarbeitung verteilt man das Reaktionsgemisch zwischen 500 ml Methylenchlorid und 500 ml gesättigter Kochsalzlösung, trocknet die organische Phase über wasserfreiem Magnesiumsulfat und dampft unter vermindertem Druck ein. Das zurückbleibende Harz (34 g) wird an 1,2 kg Kieselgel chromatographiert. Man erhält als Fraktion 1 12,6 g (eluiert mit Diäthyläther/n-Hexan 1:3) und als Fraktion 2 10,1 g (eluiert mit Diäthyläther/n-Hexan 1:1) der diastereomeren Amide der R-(-)-α-Methoxyphenylessigsäure.

Man löst die 10,1 g (26,6 mMol) der Fraktion 2 in 1,9 l Tetrahydrofuran und versetzt dann mit 41,6 g Kalium-t-butylat und 3,4 ml Wasser und erhitzt 1 Stunde zum Rückfluss. Nach dem Abkühlen wird das Gemisch zwischen Diäthyläther und 2N Natronlauge verteilt, und die organische Phase mit gesättigter Kochsalzlösung gewaschen und über wasserfreiem Magnesiumsulfat getrocknet. Nach dem Einengen unter vermindertem Druck erhält man 6,1 g (-)-1,2,3,4,4aα,5,6,10bβ-Octahydro-2α-methyl -7-methoxybenzo[f]-chinolin, welches als Hydrochlorid aus Methanol/Essigester kristallisiert, Smp. 190-193°, $[\alpha]_D$ = -85,5° (c = 1% in Methanol).

Aus Fraktion 1 erhält man in analoger Weise (+)-1,2,3,4,4aα,5,6,10bβ-Octahydro-2α-methyl -7-methoxybenzo[f]chinolin, welches als Hydrochlorid aus Methanol/Essigester kristallisiert, Smp. 193-194°, $[\alpha]_D$ = +87,4° (c = 1% in Methanol).

## Beispiel 25

5,0 g (20,5 mMol) rac-1,2,5,6-Tetrahydro-2-methyl-7-methoxybenzo[f]chinolin -3(4H)-on werden mit 3 g (27,1 mMol) Kalium-t-butylat in 80 ml t-Butylalkohol 1/4 Stunde zum Rückfluss erhitzt und nach Zugabe von 2,4 ml (20,5 mMol) Benzylchlorid weitere 3 Stunden bei dieser Temperatur gehalten. Nach dem Abkühlen verteilt man zwischen Wasser und Diäthyläther und trocknet die organische Phase über Magnesiumsulfat. Beim Eindampfen kristallisieren 5,5 g (81%) rac-1,2,5,6-Tetrahydro-2-methyl-4-benzyl -7-methoxybenzo[f]-chinolin-3-on aus, Smp. 136-137°.

5,5 g (16,5 mMol) rac-1,2,5,6-Tetrahydro-2-methyl-4-benzyl -7-methoxybenzo[f]chinolin-3-on werden in 200 ml Tetrahydrofuran mit 1,3 g Lithiumaluminiumhydrid in 100 ml trockenem Tetrahydrofuran 2 Stunden bei der Rückflusstemperatur reduziert. Nach dem Abkühlen werden bei 10° 100 ml Ammoniumchlorid zugetropft, die hydrolysierte Lösung über Kieselgur filtriert, und das Filtrat zwischen Diäthyläther und Wasser verteilt. Nach dem Trocknen der organischen Phase über Magnesiumsulfat wird unter vermindertem Druck eingedampft, wobei man 6,45 g gelbes Oel (20,2 mMol) erhält. Dieses löst man in 50 ml Acetonitril und versetzt bei 20° portionenweise mit 2,05 g (32,8 mMol) Natriumcyanborhydrid, wobei mit 6,3N Chlorwasserstoff in Aethanol (total 6,2 ml) der pH-Wert am Umschlagspunkt von Bromkresolgrün gehalten wird. Nach 16-stündiger Reaktionszeit wird überschüssiges Reaktionsmittel mit wenig konzentrierter Salzsäure zerstört, und das Gemisch anschliessend auf Eis gegossen. Mit 2N Natronlauge wird auf pH 9 gestellt und mit Diäthyläther extrahiert. Trocknen der organischen Phase über Magnesiumsulfat und Abdestillieren der flüchtigen Anteile unter vermindertem Druck führt zu einem Rohprodukt (6,3 g), welches an Kieselgel chromatographiert wird. Mit Diäthyläther/n-Hexan (1:4) eluiert man 2,6 g rac-1,2,3,4,4aα,5,6,10bβ-Octahydro-2α-methyl-4-benzyl-7-methoxybenzo[f]chinolin als hellgelbes Oel. (Hydrochlorid aus Essigester/Aethanol, Smp. 214-215°).

2,6 g (8,1 mMol) rac-1,2,3,4,4aα,5,6,10bβ-Octahydro-2α-methyl-4-benzyl -7-methoxybenzo[f]chinolin wird mit 100 ml Bromwasserstoffsäure (48%) 3 1/2 Stunden zum Rückfluss erhitzt. Nach dem Abkühlen dampft man die flüchtigen Anteile unter vermindertem Druck ab und verteilt den Rückstand zwischen gesättigter Natriumbicarbonatlösung und Methylen chlorid. Trocknen der organischen Phase über Magnesiumsulfat und Einengen liefert 2,4 g (47%) kristallines rac-1,2,3,4,4aα,5,6,10bβ-Octahydro-2α-methyl -4-benzylbenzo[f]chinolin-7-ol, Smp. 174-178°. Kristallisation aus Bromwasserstoff/Aethanol liefert das Hydrobromid, Smp. 293-294°.

## Beispiel 26

2,4 g (7,8 mMol) rac-1,2,3,4,4aα,5,6,10bβ-Octahydro-2α-methyl -4-benzylbenzo[f]chinolin-7-ol werden in 200 ml Methanol gelöst und 2 Stunden bei 20° und Atmosphärendruck in Gegenwart von 0,25 g Palladium/Kohle (10%) hydriert. Anschliessend filtriert man vom Katalysator ab, dampft das Filtrat ein und kristallisiert das Produkt aus Chlorwasserstoff/Aethanol. Man erhält auf diese Weise 1,55 g (78%) rac-1,2,3,4,4aα,5,6,10bβ-Octahydro -2α-methylbenzo[f]chinolin-7-ol-hydrochlorid, Smp. >285°.

## Beispiel 27

2,0 g (9,2 mMol) rac-1,2,3,4,4aα,5,6,10bβ-Octahydro-2α-methylbenzo[f]chinolin-7-ol werden in 100 ml Methanol gelöst, mit 0,91 ml (13,8 mMol) Acrylnitril versetzt und 17 Stunden zum Rückfluss erhitzt. Anschliessend dampft man die flüchtigen Anteile unter vermindertem Druck ab und chromatographiert den Rückstand an Kieselgel mit einem 98:2-Gemisch von Methylenchlorid und Methanol als Eluierungsmittel. Als Hauptkomponente erhält man 2,0 g rac-1,2,3,4,4aα,5,6,10bβ-Octahydro-7-hydroxy -2α-methylbenzo[f]-chinolin-4-propionitril, welche mit 5N Chlorwasserstoff in Aethanol in Hydrochlorid übergeführt werden, Smp. >312°, Ausbeute 2,1 g (74%).

## Beispiel 28

4,9 g (22,5 mMol) rac-1,2,3,4,4aα,5,6,10bβ-Octahydro-2α-methylbenzo[f]chinolin-7-ol werden in 200 ml Methanol gelöst, mit 24 g (33,8 mMol) Acrylamid versetzt und 30 Stunden zum Rückfluss erhitzt. Anschliessend dampft man die flüchtigen Anteile unter vermindertem Druck ab und chromatographiert den Rückstand an Kieselgel mit einem 92:8-Gemisch von Methylenchlorid und Methanol als Eluierungsmittel. Als Hauptkomponente erhält man 5,9 g rac-1,2,3,4,4aα,5,6,10bβ-Octahydro-7-hydroxy -2α-methylbenzo[f]-chinolin-4-propionamid, welche mit 5N Chlorwasserstoff in Aethanol in Hydrochlorid übergeführt werden, Smp. 250°, Ausbeute 4,5 g (62%).

## Beispiel 29

4,0 g (18,4 mMol) rac-1,2,3,4,4aα,5,6,10bβ-Octahydro-2α-methylbenzo[f]chinolin-7-ol werden in 200 ml Methylenchlorid gelöst und mit 5,1 ml Triäthylamin versetzt. Dazu werden 2,4 g (22,1 mMol) Methoxyessigsäurechlorid in 15 ml Methylenchlorid bei 20° getropft. Nach 20-stündigem Rühren bei 20° wird zwischen gesättigter Natriumbicarbonatlösung und Methylenchlorid verteilt, mit Methylenchlorid nachextrahiert, und die organische Phase über Magnesiumsulfat getrocknet und unter vermindertem Druck eingedampft. Der harzige Rückstand (5,6 g) wird in 60 ml trockenem Tetrahydrofuran gelöst und zu einer Suspension von 1,25 g Lithiumaluminiumhydrid in 50 ml trockenem Tetrahydrofuran getropft. Nach 1-stündigem Erhitzen zum Rückfluss wird auf 10° abgekühlt, und 20 ml gesättigte Ammoniumchloridlösung zugetropft, anschliessend 50 ml Essigester unter Rühren zugegeben und zum Schluss über Kieselgur filtriert. Nach dem Trocknen über Magnesiumsulfat und Abdestillieren der Lösungsmittel wird der Rückstand in 30 ml Aethanol gelöst und mit 10 ml 6N Chlorwasserstoff in Aethanol versetzt, wobei man 4,35 g (76%) rac-1,2,3,4,4aα,5,6,10bβ-Octahydro-4-(2-methoxyäthyl) -2α-methylbenzo[f]chinolin- 7-ol-hydrochlorid erhält, Smp. 243-245°.

## Beispiel 30

3,5 g (13 mMol) rac-1,2,3,4,4aα,5,6,10bβ-Octahydro-2α-methylbenzo[f]chinolin-7-ol werden in 150 ml Methanol gelöst, mit 1,14 g (19 mMol) Acrylsäuremethylester versetzt und 20 Stunden zum Rückfluss erhitzt. Anschliessend dampft man die flüchtigen Anteile unter vermindertem Druck ab und chromatographiert den Rückstand mit einem 98:2-Gemisch von Methylenchlorid und Methanol als Eluierungsmittel an Kieselgel. Als Hauptkomponente erhält man 4,0 g rac-1,2,3,4,4aα,5,6,10bβ-Octahydro-7-hydroxy -2α-methylbenzo[f]chinolin-4-(3-propionylmethylcarboxylat), welche mit 5N Chlorwasserstoff in Aethanol in Hydrochlorid übergeführt werden, Smp. 207-210°, Ausbeute 3,6 g (81%).

Beispiel 31

2,8 g (13 mMol) rac-1,2,3,4,4aα,5,6,10bβ-Octahydro-2α-methylbenzo[f]chinolin-7-ol in 100 ml Aethylmethylketon werden mit 5,5 g (26 mMol) 4-Chlor-4′-methoxy-butyrophenon, 3,6 g Kaliumcarbonat und 0,9 g Natriumjodid 20 Stunden zum Rückfluss erhitzt. Anschliessend kühlt man ab und verteilt zwischen Methylenchlorid und Wasser. Nach dem Trocknen über Magnesiumsulfat und Eindampfen der Lösungsmittel unter vermindertem Druck wird ein Rohprodukt erhalten, das mit Essigester als Eluierungsmittel an Kieselgel chromatographiert und als Hauptfraktion 1,9 g der Base liefert. Aus Essigester/Chlorwasserstoff/Aethanol kristallisieren 2,0 g (38%) rac-[1,2,3,4,4a,5,6,10bβ-Octahydro-2α-methyl -7-hydroxybenzo[f]chinolin-4-yl]-4′-methoxybutyrophenon -hydrochlorid, Smp. 95-97°.

Beispiel 32

4,05 g (15 mMol) rac-1,2,3,4,4aα,5,6,10bβ-Octahydro-2α-methylbenzo[f]chinolin-7-ol in 150 ml Aceton werden mit 3,6 ml (25 mMol) 4-Brombuttersäureäthylester, 5,75 g Kaliumcarbonat und 2,8 g Natriumjodid 4 Stunden zum Rückfluss erhitzt. Anschliessend kühlt man ab und verteilt zwischen Methylenchlorid und Wasser. Nach dem Trocknen über Magnesiumsulfat und Eindampfen der Lösungsmittel unter vermindertem Druck wird ein Rohprodukt erhalten, das mit Methylenchlorid/Methanol (98:2) an Kieselgel chromatographiert und als Hauptfraktion 3,65 g der Base liefert. Aus Methanol/Chlorwasserstoff/Aethanol kristallisieren 3,86 g (70%) rac-1,2,3,4,4a,5,6,10bβ-Octahydro-2α-methyl -7-hydroxybenzo[f]chinolin-4-buttersäureäthylester -hydrochlorid, Smp. 233-235°.

Beispiel 33

Zu einer Suspension von 0.58 g Lithiumaluminiumhydrid in 30 ml trockenem Tetrahydrofuran tropft man eine Lösung von 2,3 g (7,6 mMol) rac-1,2,3,4,4aα,5,6,10bβ-Octahydro-7-hydroxy -2α-methylbenzo[f]-chinolin-4-(3-propionylmethylcarboxylat) in 30 ml Tetrahydrofuran und erhitzt 1 Stunde zum Rückfluss. Nach dem Abkühlen auf 5° wird mit gesättigter Ammoniumchloridlösung hydrolysiert, mit Essigester verdünnt und filtriert. Das Filtrat wird zwischen gesättigter Natriumbicarbonatlösung und Essigester verteilt, die organische Phase über Magnesiumsulfat getrocknet und unter vermindertem Druck eingedampft. Den Rückstand löst man in 50 ml Methylenchlorid/Methanol und versetzt mit 5N Chlorwasserstoff in Aethanol. Einengen auf 15 ml Volumen liefert 11 g (46%) rac-1,2,3,4,4aα,5,6,10bβ-Octahydro-7-hydroxy -2α-methylbenzo[f]chinolin-4-propanol-hydrochlorid, Smp. 248-250°.

Beispiel 34

Zu einer Suspension von 0,7 g Natriumborhydrid in 10 ml Methanol tropft man eine Lösung von 1,4 g (3,6 mMol) rac-[1,2,3,4,4aα,5,6,10bβ-Octahydro-7-hydroxy -2α-methylbenzo[f]chinolin-4-yl]-4′-fluorbutyrophenon in 60 ml Tetrahydrofuran und rührt 1 Stunde bei 20°. Danach wird mit 100 ml Wasser hydrolysiert, mit Methylenchlorid extrahiert, und die organische Phase über Magnesiumsulfat getrocknet und unter vermindertem Druck eingedampft. Den Rückstand löst man in 40 ml Essigester und versetzt mit 5N Chlorwasserstoff in Aethanol, wobei man 0,65 g (43%) rac-1,2,3,4,4aα,5,6,10bβ-Octahydro-7-hydroxy -2α-methylbenzo[f]chinolin-4-(4-fluorphenyl)butanol -hydrochlorid erhält, Smp. 123-133°.

Beispiel 35

2,7 g (6,7 mMol) rac-1,2,3,4,4aα,5,6,10bβ-Octahydro-2α-(brommethyl) -4-phenyläthylbenzo[f]chinolin-7-ol werden in 200 ml Methanol und 20 ml Eisessig gelöst und 39 Stunden in Gegenwart von 0,3 g Palladium/Kohle (10%) bei 20° und Normaldruck hydriert. Anschliessend filtriert man vom Katalysator ab und dampft das Filtrat unter vermindertem Druck ein. Der Rückstand wird zwischen gesättigter Natriumbicarbonatlösung und Methylenchlorid verteilt, und die organische Phase über Magnesiumsulfat getrocknet und zur Trockene eingedampft. Der kristalline Rückstand wird in 100 ml Aethanol gelöst und mit 6N Chlorwasserstoff in Aethanol versetzt. Aus der Lösung kristallisieren 1,8 g (75%) weisse Kristalle von rac-1,2,3,4,4aα,5,6,10bβ-Octahydro-2α-methyl -4-phenyläthylbenzo[f]chinolin-7-ol-hydrochlorid, Smp. 285-287°.

Das als Ausgangsmaterial verwendete rac-1,2,3,4,4aα,5,6,10bβ-Octahydro-2α-(brommethyl)-4-phenyläthylbenzo[f]chinolin-7-ol wurde wie folgt hergestellt:

Man löst 8,2 g (65,4 mMol) Phenyläthylamin in 30 ml Benzol und gibt dazu bei 0-5° innerhalb von 1 Stunde eine Lösung von 9,9 g (43,6 mMol) 2-(Brommethyl)acrylsäureäthylester (85%ig) in 40 ml Benzol, welcher nach den Angaben von J. Villieras und M. Rambaud, Synthesis 1982, 924-926 hergestellt wurde. Nach 1 Stunde Rühren bei 0-5° wird eine Lösung von 6,4 g (36,3 mMol) 5-Methoxy-2-tetralon in 30 ml Benzol zugetropft und anschliessend 20 Stunden am Wasserabscheider zum Rückfluss erhitzt. Nach dem Abkühlen verteilt man zwischen Essigester und Wasser und trocknet die organische Phase über Magnesiumsulfat. Abdestillieren der flüchtigen Anteile unter vermindertem Druck ergibt 16,8 g dunkles Oel, welches mit Aether/n-Hexan (1:4) an Kieselgel chromatographiert wird. Als Hauptfraktion werden 10,05 g eines gelben Oels erhalten. Dieses (25,7 mMol) löst man in 100 ml Tetrahydrofuran und 10 ml Alkohol und gibt 3,2 g (51,4 mMol) Natriumcyanborhydrid und soviel 6,3N Chlorwasserstoff in Aethanol zu (total 5,3 ml), dass die Reaktionslösung am Umschlagspunkt von Bromcresolgrün bleibt. Nach 4-stündigem Rühren bei 20-30° wird auf Natriumbicarbonatlösung gegossen und mit Methylenchlorid extrahiert. Nach dem Waschen der organischen Phase mit Wasser wird diese über Magnesiumsulfat getrocknet und unter vermindertem Druck eingedampft. Den öligen Rückstand chromatographiert man an Kieselgel und eluiert zunächst mit einem 1:2-Gemisch von Methylenchlorid und n-Hexan, dann mit Methylenchlorid. Die nach Dünnschichtchromatographie [Laufsysteme a) Diäthyläther/n-Hexan (1:1) oder b) Methylenchlorid/Diäthyläther (9:1)] einheitlichen Fraktionen werden vereinigt und eingedampft, wobei 3,7 g (37%) rac-1,2,3,4,4aα,5,6,10bβ-Octahydro-4-phenyläthyl -7-methoxy-benzo[f]chinolin-2α-carbonsäureäthylester als orangerotes Oel erhalten werden.

3,7 g (9,4 mMol) rac-1,2,3,4,4aα,5,6,10bβ-Octahydro-4-phenyläthyl -7-methoxy-benzo[f]chinolin-2α-carbonsäureäthylester werden in 50 ml trockenem Tetrahydrofuran zu einer Suspension von 1,2 g Lithiumaluminiumhydrid in 50 ml trockenem Tetrahydrofuran getropft und anschliessend 3/4 Stunden bei 20-32° gerührt. Nach Hydrolyse der Reaktionslösung mit 50 ml Ammoniumchloridlösung bei 0-10° filtriert man über Kieselgur und extrahiert mit Essigester und Methylenchlorid. Trocknen der vereinigten organischen Extrakte über Magnesiumsulfat und Abdestillieren der Lösungsmittel unter vermindertem Druck liefern 3,25 g (98%) rac-1,2,3,4,4aα,5,6,10bβ-Octahydro-2α-(hydroxymethyl)-4-phenyläthyl-7-methoxybenzo[f]chinolin.

3,25 g (9,2 mMol) rac-1,2,3,4,4aα,5,6,10bβ-Octahydro-2α-(hydroxymethyl) -4-phenyläthyl-7-methoxybenzo[f]chinolin werden in 300 ml Bromwasserstoffsäure (48%) 4 Stunden zum Rückfluss erhitzt. Anschliessend dampft man unter vermindertem Druck die flüchtigen Anteile ab, gibt zweimal je 70 ml Toluol zu und dampft jeweils wieder ein. Der Rückstand wird zwischen gesättigter Natriumbicarbonatlösung und Methylenchlorid verteilt, und die vereinigten Extrakte über Magnesiumsulfat getrocknet und eingedampft. Den Rückstand (4,6 g) chromatographiert man an Kieselgel und erhält aus den mit Essigester eluierten Fraktionen 3,15 g (85%) hellbraune Kristalle von rac-1,2,3,4,4aα,5,6,10bβ-Octahydro-2α-(brommethyl) -4-phenyläthylbenzo[f]chinolin-7-ol. Umkristallisation aus Essigester liefert beige Kristalle vom Schmelzpunkt 160-162°.

## Beispiel 36

5,2 g (20 mMol) rac-1,2,3,4,4aα,5,6,10bβ-Octahydro-2α-methyl -4-propylbenzo[f]chinolin-7-ol werden in 100 ml Methanol und 100 ml Methylenchlorid gelöst und mit 3,85 g (11,1 mMol) (+)-2,2′-(1,1′-Binaphthyl)-phosphorsäure in 100 ml Methanol und 100 ml Methylenchlorid versetzt und unter vermindertem Druck auf 100 ml Volumen eingedampft. Aus der Lösung kristallisieren dann 6,3 g beige Kristalle, welche dreimal aus Aethanol/Methanol/Chloroform (1:1:1) umkristallisiert werden. Die verbleibenden 2,3 g Kristalle (Smp. >300°) werden zwischen Diäthyläther und verdünnter Ammoniaklösung verteilt, die organische Phase über Magnesiumsulfat getrocknet und eingedampft. 0,8 g der optischen aktiven Base (Smp. 178-187°) wird mit Chlorwasserstoff in Aethanol ins Hydrochlorid übergeführt. Man erhält so 0,7 g (25%) weisse Kristalle von

(+)-1,2,3,4,4aα,5,6,10bβ-Octahydro-2α-methyl -4-propylbenzo[f]chinolin-7-ol-hydrochlorid, Smp. 275-277°, $[\alpha]_D$ = +67,4° (c=1% in Methanol).

Alle Mutterlaugen aus den oben beschriebenen Kristallisationen werden zwischen Diäthyläther und verdünnter Ammoniaklösung verteilt, die organische Phase über Magnesiumsulfat getrocknet und eingedampft. Der Rückstand (3,75 g Base) wird in 200 ml Methylenchlorid/Methanol (1:1) gelöst und mit 4,52 g (13 mMol) (-)-2,2′-(1,1′-Binaphthyl)phosphorsäure in 200 ml Methylenchlorid/Methanol (1:1) versetzt. Nach Einengen unter vermindertem Druck auf 140 ml Volumen kristallisieren 6,9 g beige Kristalle aus, welche dreimal aus Aethanol/Methanol/Chloroform (1:1:1) umkristallisiert werden. Man erhält schliesslich 3,6 g Kristalle (Smp. >300°), welche zwischen Diäthyläther und verdünnter Ammoniaklösung verteilt werden. Aus der organischen Phase erhält man nach Trocknen über Magnesiumsulfat, Filtrieren und Einengen 1,5 g der optisch aktiven Base (Smp. 176-187°), welche mit Chlorwasserstoff in Aethanol ins Hydrochlorid übergeführt werden, wobei man 1,45 g (52%) weisse Kristalle von (-)-1,2,3,4,4aα,5,6,10bβ-Octahydro-2α-methyl -4-propylbenzo[f]chinolin-7-ol-hydrochlorid, Smp. 275-277°, $[\alpha]_D$ = -68,4° (c=1% in Methanol) erhält.

## Beispiel 37

Eine Suspension von 2,7 g (9,1 mMol) rac-1,2,3,4,4aα,5,6,10bβ-Octahydro-2α-methyl -4-propylbenzo[f]-chinolin-7-ol-hydrochlorid in 50 ml Methylenchlorid und 8,4 ml (60 mMol) Triäthylamin wird mit 1,76 ml (22 mMol) Pivalinsäurechlorid in 10 ml Methylenchlorid bei 20° versetzt und 15 Stunden gerührt. Anschliessend verteilt man die Reak tionslösung zwischen gesättigter Natriumbicarbonatlösung und Methylenchlorid und stellt den pH-Wert der wässrigen Phase mit 2N Natronlauge auf 8 ein. Nach dem Trocknen der organischen Phase über Magnesiumsulfat wird eingedampft, und der Rückstand an Kieselgel chromatographiert. Mit Methylenchlorid/Methanol (98:2) eluiert man 3,6 g öliges Produkt, das aus Methanol/Essigester nach Zugabe von Chlorwasserstoff in Aethanol als Hydrochlorid kristallisiert. Auf diese Weise erhält man 3,15 g (91%) rac-1,2,3,4,4aα,5,6,10bβ-Octahydro-2α-methyl -4-propylbenzo[f]chinolin-7-yl-pivalat-hydrochlorid, Smp. 222-224°.

## Beispiel 38

3,05 g (16 mMol) (-)-1,2,3,4,4aα,5,6,10bβ-Octahydro-2α-methyl -7-methoxybenzo[f]chinolin in 35 ml Methylenchlorid und 4,7 ml Triäthylamin werden unter Rühren bei 20° mit 2 g (19 mMol) Methoxyessigsäurechlorid in 15 ml Methylenchlorid versetzt. Nach 18 Stunden bei 20° wird zwischen Methylenchlorid und Natriumbicarbonatlösung verteilt, und die organische Phase über Magnesiumsulfat getrocknet und unter vermindertem Druck eingedampft. Das zurückbleibende Oel (5,2 g) wird an Kieselgel chromatographiert.

Eluieren mit Essigester/n-Hexan (1:2) liefert die Hauptfraktion (3,1 g), welche in 55 ml trockenem Tetrahydrofuran gelöst und mit 0,8 g Lithiumaluminiumhydrid reduziert wird (1-stündiges Erhitzen zum Rückfluss). Zur Aufarbeitung hydrolysiert man mit 10 ml gesättigter Ammoniumchloridlösung, verdünnt mit 50 ml Essigester und filtriert durch Kieselgur. Nach dem Trocknen der organischen Phase über Magnesiumsulfat wird eingedampft, und der Rückstand an Kieselgel chromatographiert. Mit Methylenchlorid/Methanol (99:1) eluiert man als Hauptfraktion 2,3 g Oel, welche in 90 ml Bromwasserstoffsäure (48%) gelöst und 1 Stunde zum Rückfluss erhitzt werden. Anschliessend werden die flüchtigen Bestandteile unter vermindertem Druck abdestilliert, und der Rückstand zwischen Methylenchlorid und verdünnter Natronlauge bei pH 8 verteilt. Die organische Phase wird über Magnesiumsulfat getrocknet und eingedampft, und der Rückstand in Methanol mit Chlorwasserstoff versetzt und durch Zugabe von Essigester zur Kristallisation gebracht. Man erhält so 1,5 g (32%) beige Kristalle von (-)-1 ,2,3,4,4aα,5,6,10bβ-Octahydro-2α-methyl-7-hydroxybenzo[f]chinolin-4-äthanol-hydrochlorid, Smp. 239-241°, $[\alpha]_D$ = -67,1° (c=1% in Methanol).

## Beispiel 39

10 g (43 mMol) rac-1,2,3,4,4aα,5,6,10bβ-Octahydro-2α-methyl -7-methoxybenzo[f]chinolin in 300 ml Aethylmethylkton werden mit 8 ml 2-Chlorcyclopentanon 11 g Kaliumcarbonat und 2 g Natriumjodid 20

Stunden zum Rückfluss erhitzt. Anschliessend destilliert man die flüchtigen Anteile unter verminderten Druck ab und verteilt den Rückstand zwischen Wasser und Methylenchlorid. Der Rückstand aus der organischen Phase wird mit Methylenchlorid/Methanol (99:1) als Eluierungsmittel an Kieselgel chromatographiert, wobei 12,4 g (92%) rac-1,2,3,4,4aα,5,6,10bβ-Octahydro-2α-methyl-4-(2-cyclopentanonyl)-7-methoxybenzo[f]chinolin erhalten werden, Smp. 126-128°

3 g (9,4 mMol) rac-1,2,3,4,4aα,5,6,10bβ-Octahydro-2α-methyl -4-(2-cyclopentanonyl)-7-methoxybenzo-[f]chinolin werden mit 1,5 ml Hydrazinhydrat, 2,24 g pulverisiertem Kaliumhydroxid und 10 ml Triäthylenglycol 2 Stunden zum Rückfluss erhitzt (Innentemperatur 214°). Danach bestückt man den Kolben mit einem absteigenden Kühler, destilliert langsam ein Gemisch von Hydrazin und Wasser ab und hält das Reaktionsgemisch für weitere 2 Stunden bei 195° Kolbeninnentemperatur. Nach dem Abkühlen wird auf Wasser gegossen und mit Methylenchlorid extrahiert. Der Rückstand aus der organischen Phase wird mit Methylenchlorid/Methanol (19:1) als Eluierungsmittel an Kieselgel chromatographiert, und die erhaltenen 1,6 g Base (56%) mit Chlorwasserstoff in Aethanol ins Hydrochlorid über geführt wobei 1,5 g rac-1,2,3,4,4aα,5,6,10bβ-Octahydro-4-cyclopentyl-2α-methyl[f]chinolin-7-ol-hydrochlorid auskristallisieren, Smp. >310°.

## Beispiel 40

8,0 g (34,5 mMol) (-)-1,2,3,4,4aα,5,6,10bβ-Octahydro-2α-methyl -7-methoxybenzo[f]chinolin werden in 250 ml Bromwasserstoffsäure (48%) unter Rühren in einer Argonatmosphäre 1 Stunde zum Rückfluss erhitzt. Nach dem Abkühlen destilliert man die überschüssige Bromwasserstoffsäure unter vermindertem Druck ab und verteilt den Rückstand zwischen Methylenchlorid/Methanol (5:1) und gesättigter wässriger Natriumbicarbonatlösung. Die wässrige Phase wird mit konz. Natronlauge auf pH 9 gestellt, und die organische Phase abgetrennt. Nach dreimaliger Extraktion der wässrigen Phase mit Methylenchlorid werden die organischen Extrakte vereinigt, über wasserfreiem Magnesiumsulfat getrocknet und unter vermindertem Druck eingedampft. Der kristalline Rückstand (7,0 g; 93%) wird in 150 ml Methanol gelöst, filtriert und mit Chlorwasserstoff in Aethanol angesäuert. Beim Einengen der Lösung auf 75 ml Volumen kristallisiert das (-)-1,2,3,4,4aα,5,6,10bβ-Octahydro-2α-methyl-benzo[f]chinolin-7-ol-hydrochlorid aus, Smp. 300-305°, $[\alpha]_D$ = -86,0° (c = 1% in Methanol).

| Beispiel A | |
|---|---|
| Tablette à 10 mg | |
| Zusammensetzung | |
| 1. (-)-1,2,3,4,4a$\alpha$,5,6,10b$\beta$-Octahydro-2$\alpha$-methyl-7-hydroxybenzo[f]chinolin-4-äthanol-hydrochlorid | 11,38 mg *) |
| 2. Milchzucker pulv. | 98,62 mg |
| 3. Maisstärke | 45,00 mg |
| 4. Polyvinylpyrrolidon K 30 | 15,00 mg |
| 5. Maisstärke | 25,00 mg |
| 6. Talk | 4,50 mg |
| 7. Magnesiumstearat | 0,50 mg |
| Tablettengewicht | 200,00 mg |

*) entspricht 10 mg Base

Verfahren:

1-3 werden gemischt und durch ein Sieb mit 0,5 mm Maschenweite gesiebt. Diese Pulvermischung wird mit einer alkoholischen Lösung von 4 befeuchtet und geknetet. Die feuchte Mase wird granuliert, getrocknet und auf eine geeignete Korngrösse zugerüstet. Dem getrockneten Granulat werden nacheinander 5, 6 und 7 zugefügt und gemischt. Die pressfertige Mischung wird zu Tabletten geeigneter Grösse mit einem Sollgewicht von 200 mg verpresst.

| Beispiel B | |
|---|---|
| Gelatinesteck-Kapseln à 20 mg | |
| Zusammensetzung | |
| 1. (-)-1,2,3,4,4aα,5,6,10bβ-Octahydro-2α-methyl-7-hydroxybenzo[f]chinolin-4-äthanol-hydrochlorid | 22,76 mg *) |
| 2. Milchzucker pulv. | 63,24 mg |
| 3. Maisstärke | 40,00 mg |
| 4. Talk | 3,60 mg |
| 5. Magnesiumstearat | 0,40 mg |
| 6. Milchzucker krist. | 110,00 mg |
| Kapselfüllgewicht | 240,00 mg |

*) entspricht 20 mg Base

Verfahren:

1-5 werden gemischt und durch ein Sieb mit 0,5 mm Maschenweite gesiebt. Danach wird 6 zugefügt und gemischt. Diese abfüllfertige Mischung wird in Gelatinesteckkapseln geeigneter Grösse (z.B. Nr. 2) mit einem Einzelfüllgewicht von 240 mg abgefüllt.

Beispiel C

Wenn man nach den in den Beispielen A und B beschriebenen Verfahren arbeitet, können aus den folgenden, ebenfalls bevorzugten Verbindungen entsprechende Tabletten bzw. Kapseln hergestellt werden:
rac-1,2,3,4,4aα,5,6,10bβ-Octahydro-2α-methyl-4-propylbenzo[f]chinolin-7-ol,
(-)-1,2,3,4,4aα,5,6,10bβ-Octahydro-2α-methyl-4-propylbenzo[f]chinolin-7-ol,
rac-1,2,3,4,4aα,5,6,10bβ-Octahydro-4-isopropyl-2α-methylbenzo[f]chinolin-7-ol,
rac-1,2,3,4,4aα,5,6,10bβ-Octahydro-4-cyclopentyl-2α-methylbenzo[f]chinolin-7-ol,
(-)-1,2,3,4,4aα,5,6,10bβ-Octahydro-4-cyclopentyl-2α-methylbenzo[f]chinolin-7-ol,
rac-1,2,3,4,4aα,5,6,10bβ-Octahydro-7-hydroxy-2α-methylbenzo[f]chinolin-4-äthanol und
(-)-1,2,3,4,4aα,5,6,10bβ-Octahydro-2α-methylbenzo[f]chinolin-7-ol.

## Ansprüche

1. Octahydrobenzo[f]chinoline der allgemeinen Formel

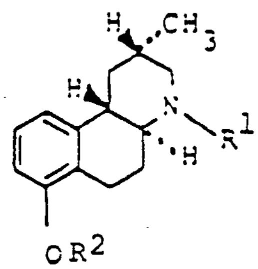

I

worin $R^1$ Wasserstoff, nieder-Alkyl, welches gegebenenfalls durch Hydroxy, nieder-Alkoxy, Cyan, Halogen, nieder-Alkoxycarbonyl, Aminocarbonyl, substituiertes Benzoyl oder substituiertes α-Hydroxybenzyl substituiert ist, nieder-Alkenyl, Cycloalkyl, Cycloalkyl-nieder-alkyl, Heteroaryl-nieder-alkyl oder gegebenenfalls durch Hydroxy, nieder-Alkoxy, nieder-Alkyl oder Halogen substituiertes Aryl-nieder-alkyl und $R^2$ Wasserstoff oder nieder-Alkanoyl bedeuten,
in Form der Racemate und der optischen Antipoden sowie pharmazeutisch verwendbare Säureadditionssalze davon.

2. Verbindungen gemäss Anspruch 1, worin $R^2$ Wasserstoff oder Acetyl bedeutet.

3. Verbindungen gemäss Anspruch 2, worin $R^2$ Wasserstoff bedeutet.

4. Verbindungen gemäss einem der Ansprüche 1-3, worin $R^1$ Wasserstoff, nieder-Alkyl, welches gegebenenfalls durch Hydroxy, Cyan oder substituiertes Benzoyl substituiert ist, Cycloalkyl, Cycloalkylalkyl oder gegebenenfalls durch Hydroxy substituiertes Aryl-nieder-alkyl bedeutet.

5. Verbindungen gemäss Anspruch 4, worin $R^1$ Wasserstoff, nieder-Alkyl, welches gegebenenfalls durch Hydroxy, Cyan oder 4-Methoxybenzoyl substituiert ist, Cyclopentyl, Cyclopentyläthyl oder 4-Hydroxyphenäthyl bedeutet.

6. Verbindungen gemäss Anspruch 5, worin es sich bei der nieder-Alkylgruppe um Aethyl, Propyl oder Isopropyl handelt.

7. Verbindungen gemäss einem der Ansprüche 1-6, worin $R^1$ Wasserstoff, gegebenenfalls durch Hydroxy, Cyan oder 4-Methoxybenzoyl substituiertes Aethyl, Propyl oder Isopropyl, Cyclopentyl, Cyclopentyläthyl oder 4-Hydroxyphenäthyl und $R^2$ Wasserstoff bedeuten.

8. rac-1,2,3,4,4aα,5,6,10bβ-Octahydro-2α-methyl-4-propylbenzo[f]chinolin-7-ol, rac-1,2,3,4,4aα,5,6,10bβ-Octahydro-4-(4-hydroxyphenäthyl)-2α-methylbenzo[f]chinolin-7-ol, rac-1,2,3,4,4aα,5,6,10bβ-Octahydro-7-hydroxy-2α-methylbenzo[f]chinolin-4-äthanol, rac-1,2,3,4,4aα,5,6,10bβ-Octahydro-7-hydroxy-2α-methylbenzo[f]chinolin-4-butyronitril, rac-1,2,3,4,4aα,5,6,10bβ-Octahydro-4-cyclopentyl-2α-methylbenzo[f]-chinolin-7-ol, rac-1,2,3,4,4aα,5,6,10bβ-Octahydro-4-isopropyl-2α-methylbenzo[f]chinolin-7-ol, rac-1,2,3,4,4aα,5,6,10bβ-Octahydro-4-cyclopentyläthyl-2α-methylbenzo[f]chinolin-7-ol, rac-[1,2,3,4,4aα,5,6,10bβ-Octahydro-7-hydroxy-2α-methylbenzo[f]chinolin-4-yl]-4′-methoxybutyrophenon oder rac-1,2,3,4,4aα,5,6,10bβ-Octahydro-2α-methylbenzo[f]chinolin7-ol.

9. (-)-1,2,3,4,4aα,5,6,10bβ-Octahydro-4-cyclopentyl-2α-methylbenzo[f]chinolin-7-ol.

10. (-)-1,2,3,4,4aα,5,6,10bβ-Octahydro-2α-methyl-4-propylbenzo[f]chinolin-7-ol.

11. (-)-1,2,3,4,4aα,5,6,10bβ-Octahydro-7-hydroxy-2α-methylbenzo[f]chinolin-4-äthanol.

12. (-)-1,2,3,4,4aα,5,6,10bβ-Octahydro-2α-methylbenzo[f]chinolin-7-ol.

13. Verbindungen der allgemeinen Formeln

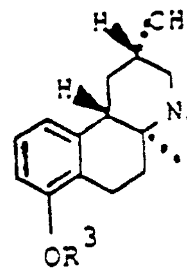

II

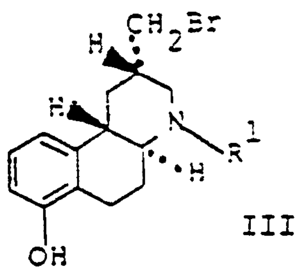

III

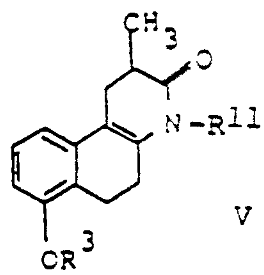

V

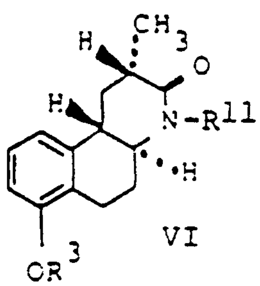

VI

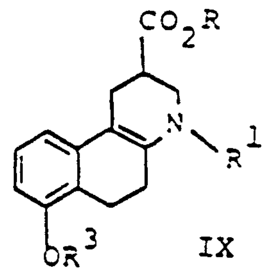

IX

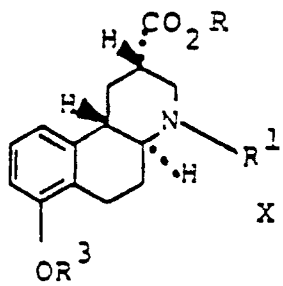

X

und

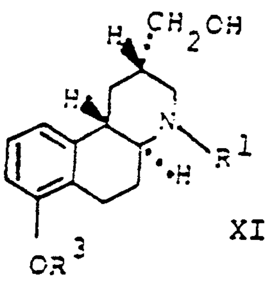

XI

worin R nieder-Alkyl, R' Wasserstoff, nieder-Alkyl, welches gegebenenfalls durch Hydroxy, nieder-Alkoxy, Cyan, Halogen, nieder-Alkoxycarbonyl, Aminocarbonyl, substituiertes Benzoyl oder substituiertes α-Hydroxybenzyl substituiert ist, nieder-Alkenyl, Cycloalkyl, Cycloalkyl-nieder-alkyl, Heteroaryl-nieder-alkyl oder gegebenenfalls durch Hydroxy, nieder-Alkoxy, nieder-Alkyl oder Halogen substituiertes Aryl-nieder-alkyl, $R^3$ nieder-Alkyl und $R^{11}$ Wasserstoff oder Benzyl bedeuten.

14. Octahydrobenzo[f]chinoline gemäss einem der Ansprüche 1-12 zur Anwendung als therapeutische Wirkstoffe.

15. Octahydrobenzo[f]chinoline gemäss einem der Ansprüche 1-12 zur Anwendung bei der Bekämpfung bzw. Verhütung von psychotischen Störungen, insbesondere chronischer Schizophrenie.

16. Verfahren zur Herstellung einer Verbindung gemäss einem der Ansprüche 1-12, dadurch gekennzeichnet, dass man

a) zur Herstellung einer Verbindung der Formel I, worin $R^2$ Wasserstoff bedeutet, in einer Verbindung der allgemeinen Formel

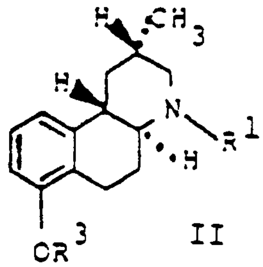

II

worin $R^3$ nieder-Alkyl bedeutet und $R^1$ die in Anspruch 1 angegebene Bedeutung besitzt, die Aethergruppe spaltet, oder

b) zur Herstellung einer Verbindung der Formel I, worin $R^2$ Wasserstoff bedeutet, eine Verbindung der allgemeinen Formel

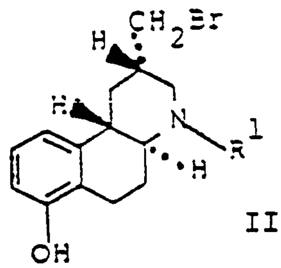

III

worin $R^1$ die in Anspruch 1 angegebene Bedeutung besitzt, reduktiv dehalogeniert, oder

c) zur Herstellung einer Verbindung der Formel I, worin $R^1$ nieder-Alkyl, welches gegebenenfalls durch Hydroxy, nieder-Alkoxy, Cyan, Halogen, nieder-Alkoxycarbonyl, Aminocarbonyl, substituiertes Benzoyl oder substituiertes α-Hydroxybenzyl substituiert ist, nieder-Alkenyl, Cycloalkyl, Cycloalkyl-nieder-alkyl, Heteroaryl-nieder-alkyl oder gegebenenfalls durch Hydroxy, nieder-Alkoxy, nieder-Alkyl oder Halogen substituiertes Aryl-nieder-alkyl und $R^2$ Wasserstoff bedeuten, eine Verbindung der Formel I, worin $R^1$ und $R^2$ je Wasserstoff bedeuten, am Stickstoffatom entsprechend substituiert, oder

d) zur Herstellung einer Verbindung der Formel I, worin $R^2$ nieder-Alkanoyl bedeutet, eine Verbindung der Formel I, worin $R^2$ Wasserstoff bedeutet, O-acyliert, oder

e) zur Herstellung einer Verbindung der Formel I, worin $R^1$ durch Hydroxy oder substituiertes α-Hydroxybenzyl substituiertes nieder-Alkyl und $R^2$ Wasserstoff bedeuten, eine Verbindung der Formel I, worin $R^1$ durch nieder-Alkoxycarbonyl oder substituiertes Benzoyl substituiertes nieder-Alkyl und $R^2$ Wasserstoff bedeuten, reduziert, oder

f) zur Herstellung einer Verbindung der Formel I, worin $R^1$ und $R^2$ je Wasserstoff bedeuten, eine Verbindung der Formel I, worin $R^1$ Benzyl und $R^2$ Wasserstoff bedeuten, debenzy liert, und

g) erwünschtenfalls ein erhaltenes Racemat in die optischen Antipoden auftrennt, und/oder

h) erwünschtenfalls eine erhaltene Verbindung in ein pharmazeutisch verwendbares Säureadditionssalz überführt.

17. Arzneimittel, enthaltend ein Octahydrobenzo[f]chinolin gemäss einem der Ansprüche 1-12 und ein therapeutisch inertes Excipiens.

18. Mittel zur Bekämpfung bzw. Verhütung von psychotischen Störungen, insbesondere von chronischer Schizophrenie, enthaltend ein Octahydrobenzo[f]chinolin gemäss einem der Ansprüche 1-12 und ein therapeutisch inertes Excipiens.

19. Verwendung eines Octahydrobenzo[f]chinolins gemäss einem der Ansprüche 1-12 bei der Bekämpfung bzw. Verhütung von Krankheiten.

20. Verwendung eines Octahydrobenzo[f]chinolins gemäss einem der Ansprüche 1-12 bei der Bekämpfung bzw. Verhütung von psychotischen Störungen, insbesondere von chronischer Schizophrenie.

21. Verwendung eines Octahydrobenzo[f]chinolins gemäss einem der Ansprüche 1-12 zur Herstellung von Mitteln gegen psychotische Störungen, insbesondere gegen chronische Schizophrenie.

Patentansprüche für folgenden Vertragsstaat: GR

1. Verfahren zur Herstellung von Octahydrobenzo[f]chinolinen der allgemeinen Formel

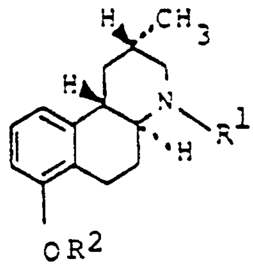

I

worin $R^1$ Wasserstoff, nieder-Alkyl, welches gegebenenfalls durch Hydroxy, nieder-Alkoxy, Cyan, Halogen, nieder-Alkoxycarbonyl, Aminocarbonyl, substituiertes Benzoyl oder substituiertes $\alpha$-Hydroxybenzyl substituiert ist, nieder-Alkenyl, Cycloalkyl, Cycloalkyl-nieder-alkyl, Heteroaryl-nieder-alkyl oder gegebenenfalls durch Hydroxy, nieder-Alkoxy, nieder-Alkyl oder Halogen substituiertes Aryl-nieder-alkyl und $R^2$ Wasserstoff oder nieder-Alkanoyl bedeuten,
in Form der Racemate und der optischen Antipoden sowie pharmazeutisch verwendbaren Säureadditionssalzen davon, dadurch gekennzeichnet, dass man

a) zur Herstellung einer Verbindung der Formel I, worin $R^2$ Wasserstoff bedeutet, in einer Verbindung der allgemeinen Formel

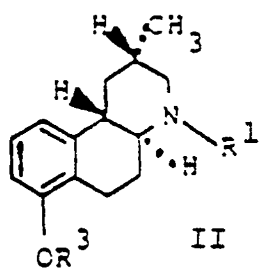

II

worin $R^3$ nieder-Alkyl bedeutet und $R'$ die oben angegebene Bedeutung besitzt,
die Aethergruppe spaltet, oder

b) zur Herstellung einer Verbindung der Formel I, worin $R^2$ Wasserstoff bedeutet, eine Verbindung der allgemeinen Formel

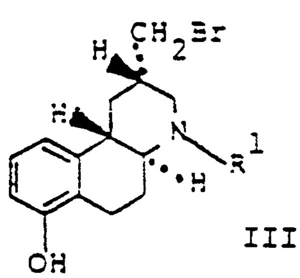

III

worin $R^1$ die oben angegebene Bedeutung besitzt,
reduktiv dehalogeniert, oder

c) zur Herstellung einer Verbindung der Formel I, worin $R^1$ nieder-Alkyl, welches gegebenenfalls durch Hydroxy, nieder-Alkoxy, Cyan, Halogen, nieder-Alkoxycarbonyl, Aminocarbonyl, substituiertes Benzoyl oder substituiertes $\alpha$-Hydroxybenzyl substituiert ist, nieder-Alkenyl, Cycloalkyl, Cycloalkyl-nieder-alkyl, Heteroaryl-nieder-alkyl oder gegebenenfalls durch Hydroxy, nieder-Alkoxy, nieder-Alkyl oder Halogen substituiertes Aryl-nieder-alkyl und $R^2$ Wasserstoff bedeuten, eine Verbindung der Formel I, worin $R'$ und $R^2$ je Wasserstoff bedeuten, am Stickstoffatom entsprechend substituiert, oder

d) zur Herstellung einer Verbindung der Formel I, worin $R^2$ nieder-Alkanoyl bedeutet, eine Verbindung der Formel I, worin $R^2$ Wasserstoff bedeutet, O-acyliert, oder

e) zur Herstellung einer Verbindung der Formel I, worin $R^1$ durch Hydroxy oder substituiertes α-Hydroxybenzyl substituiertes nieder-Alkyl und $R^2$ Wasserstoff bedeuten, eine Verbindung der Formel I, worin $R^1$ durch nieder-Alkoxycarbonyl oder substituiertes Benzoyl substituiertes nieder-Alkyl und $R^2$ Wasserstoff bedeuten, reduziert, oder

f) zur Herstellung einer Verbindung der Formel I, worin $R^1$ und $R^2$ je Wasserstoff bedeuten, eine Verbindung der Formel I, worin $R^1$ Benzyl und $R^2$ Wasserstoff bedeuten, debenzyliert, und

g) erwünschtenfalls ein erhaltenes Racemat in die optischen Antipoden auftrennt, und/oder

h) erwünschtenfalls eine erhaltene Verbindung in ein pharmazeutisch verwendbares Säureadditionssalz überführt.

2. Verfahren gemäss Anspruch 1, worin $R^2$ Wasserstoff oder Acetyl bedeutet.

3. Verfahren gemäss Anspruch 2, worin $R^2$ Wasserstoff bedeutet.

4. Verfahren gemäss einem der Ansprüche 1-3, worin $R^1$ Wasserstoff, nieder-Alkyl, welches gegebenenfalls durch Hydroxy, Cyan oder substituiertes Benzoyl substituiert ist, Cycloalkyl, Cycloalkylalkyl oder gegebenenfalls durch Hydroxy substituiertes Aryl-nieder-alkyl bedeutet.

5. Verfahren gemäss Anspruch 4, worin $R^1$ Wasserstoff, nieder-Alkyl, welches gegebenenfalls durch Hydroxy, Cyan oder 4-Methoxybenzoyl substituiert ist, Cyclopentyl, Cyclopentyläthyl oder 4-Hydroxyphenäthyl bedeutet.

6. Verfahren gemäss Anspruch 5, worin es sich bei der nieder-Alkylgruppe um Aethyl, Propyl oder Isopropyl handelt.

7. Verfahren gemäss einem der Ansprüche 1-6, worin $R^1$ Wasserstoff, gegebenenfalls durch Hydroxy, Cyan oder 4-Methoxybenzoyl substituiertes Aethyl, Propyl oder Isopropyl, Cyclopentyl, Cyclopentyläthyl oder 4-Hydroxyphenäthyl und $R^2$ Wasserstoff bedeuten.

8. Verfahren gemäss Anspruch 1, dadurch gekennzeichnet, dass man rac-1,2,3,4,4aα,5,6,10bβ-Octahydro-2α-methyl-4-propylbenzo[f]chinolin-7-ol, rac-1,2,3,4,4aα,5,6,10bβ-Octahydro-4-(4-hydroxyphenäthyl)-2α-methylbenzo[f]chinolin-7-ol, rac-1,2,3,4,4aα,5,6,10bβ-Octahydro-7-hydroxy-2α-methylbenzo[f]-chinolin-4-äthanol, rac-1,2,3,4,4aα,5,6,10bβ-Octahydro-7-hydroxy-2α-methylbenzo[f]chinolin-4-butyronitril, rac-1,2,3,4,4aα,5,6,10bβ-Octahydro-4-cyclopentyl-2α-methylbenzo[f]chinolin-7-ol, rac-1,2,3,4,4aα,5,6,10bβ-Octahydro-4-isopropyl-2α-methylbenzo[f]chinolin-7-ol, rac-1,2,3,4,4aα,5,6,10bβ-Octahydro-4-cyclopentyläthyl-2α-methylbenzo[f]chinolin-7-ol, rac-[1,2,3,4,4aα,5,6,10bβ-Octahydro-7-hydroxy-2α-methylbenzo[f]chinolin-4-yl]-4′-methoxybutyrophenon oder rac-1,2,3,4,4aα,5,6,10bβ-Octahydro-2α-methylbenzo[f]chinolin-7-ol herstellt.

9. Verfahren gemäss Anspruch 1, dadurch gekennzeichnet, dass man (-)-1,2,3,4,4aα,5,6,10bβ-Octahydro-4-cyclopentyl-2α-methylbenzo[f]chinolin-7-ol herstellt.

10. Verfahren gemäss Anspruch 1, dadurch gekennzeichnet, dass man (-)-1,2,3,4,4aα,5,6,10bβ-Octahydro-2α-methyl-4-propylbenzo[f]chinolin-7-ol herstellt.

11. Verfahren gemäss Anspruch 1, dadurch gekennzeichnet, dass man (-)-1,2,3,4,4aα,5,6,10bβ-Octahydro-7-hydroxy-2α-methylbenzo[f]chinolin-4-äthanol herstellt.

12. Verfahren gemäss Anspruch 1, dadurch gekennzeichnet, dass man (-)-1,2,3,4,4aα,5,6,10bβ-Octahydro-2α-methylbenzo[f]chinolin-7-ol herstellt.

13, Verfahren zur Herstellung von Arzneimitteln, insbesondere zur Verwendung bei der Bekämpfung bzw. Verhütung von psychotischen Störungen, insbesondee von chronischer Schizophrenie, dadurch gekennzeichnet, dass man ein Octahydrobenzo[f]chinolin der Formel I in Anspruch 1, in Form des Racemates oder eines optischen Antipoden, oder ein pharmazeutisch verwendbares Säureadditionssalz davon in eine galenische Darreichungsform bringt.

14. Verwendung eines Octahydrobenzo[f]chinolins der Formel I in Anspruch 1, in Form eines Racemates oder eines optischen Antipoden, oder eines pharmazeutisch verwendbaren Säureadditionssalzes davon zur Herstellung von Mitteln gegen psychotische Störungen, insbesondere gegen chronische Schizophrenie.

15. Verbindungen der allgemeinen Formeln

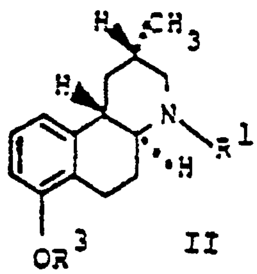

II

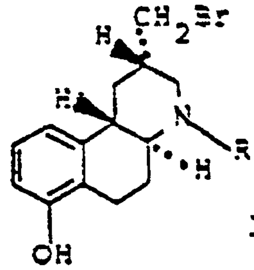

III

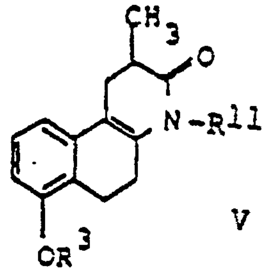

V

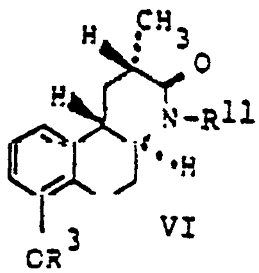

VI

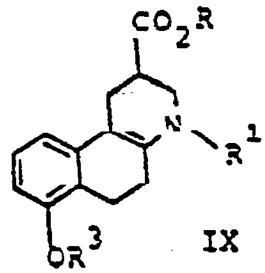

IX

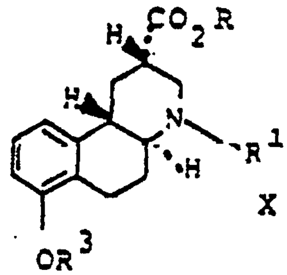

X

und

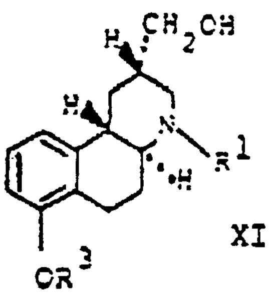

XI

worin R nieder-Alkyl, R' Wasserstoff, nieder-Alkyl, welches gegebenenfalls durch Hydroxy, nieder-Alkoxy, Cyan, Halogen, nieder-Alkoxycarbonyl, Aminocarbonyl, substituiertes Benzoyl oder substituiertes α-Hydroxybenzyl substituiert ist, nieder-Alkenyl, Cycloalkyl, Cycloalkyl-nieder-alkyl, Heteroaryl-nieder-alkyl oder gegebenenfalls durch Hydroxy, nieder-Alkoxy, nieder-Alkyl oder Halogen substituiertes Aryl-nieder-alkyl, $R^3$ nieder-Alkyl und R'' Wasserstoff oder Benzyl bedeuten.

Patentansprüche für folgenden Vertragsstaat: ES

1. Verfahren zur Herstellung von Octahydrobenzo[f]chinolinen der allgemeinen Formel

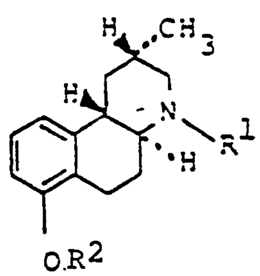

I

worin $R^1$ Wasserstoff, nieder-Alkyl, welches gegebenenfalls durch Hydroxy, nieder-Alkoxy, Cyan, Halogen, nieder-Alkoxycarbonyl, Aminocarbonyl, substituiertes Benzoyl oder substituiertes α-Hydroxybenzyl substituiert ist, nieder-Alkenyl, Cycloalkyl, Cycloalkyl-nieder-alkyl, Heteroaryl-nieder-alkyl oder gegebenenfalls durch Hydroxy, nieder-Alkoxy, nieder-Alkyl oder Halogen substituiertes Aryl-nieder-alkyl und $R^2$ Wasserstoff oder nieder-Alkanoyl bedeuten,
in Form der Racemate und der optischen Antipoden sowie pharmazeutisch verwendbaren Säureadditionssalzen davon, dadurch gekennzeichnet, dass man

a) zur Herstellung einer Verbindung der Formel I, worin $R^2$ Wasserstoff bedeutet, in einer Verbindung der allgemeinen Formel

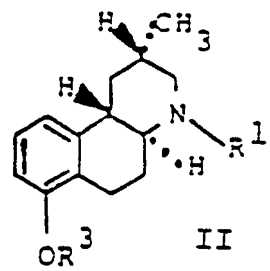

II

worin $R^3$ nieder-Alkyl bedeutet und $R^1$ die oben angegebene Bedeutung besitzt, die Aethergruppe spaltet, oder

b) zur Herstellung einer Verbindung der Formel I, worin $R^2$ Wasserstoff bedeutet, eine Verbindung der allgemeinen Formel

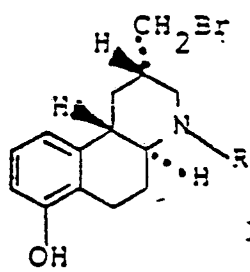

III

worin $R^1$ die oben angegebene Bedeutung besitzt, reduktiv dehalogeniert, oder

c) zur Herstellung einer Verbindung der Formel I, worin $R^1$ nieder-Alkyl, welches gegebenenfalls durch Hydroxy, nieder-Alkoxy, Cyan, Halogen, nieder-Alkoxycarbonyl, Aminocarbonyl, substituiertes Benzoyl oder substituiertes α-Hydroxybenzyl substituiert ist, nieder-Alkenyl, Cycloalkyl, Cycloalkyl-nieder-alkyl, Heteroaryl-nieder-alkyl oder gegebenenfalls durch Hydroxy, nieder-Alkoxy, nieder-Alkyl oder Halogen substituiertes Aryl-nieder-alkyl und $R^2$ Wasserstoff bedeuten, eine Verbindung der Formel I, worin $R^1$ und $R^2$ je Wasserstoff bedeuten, am Stickstoffatom entsprechend substituiert, oder

d) zur Herstellung einer Verbindung der Formel I, worin $R^2$ nieder-Alkanoyl bedeutet, eine Verbindung der Formel I, worin $R^2$ Wasserstoff bedeutet, O-acyliert, oder

e) zur Herstellung einer Verbindung der Formel I, worin $R^1$ durch Hydroxy oder substituiertes α-Hydroxybenzyl substituiertes nieder-Alkyl und $R^2$ Wasserstoff bedeuten, eine Verbindung der Formel I, worin $R^1$ durch nieder-Alkoxycarbonyl oder substituiertes Benzoyl substituiertes nieder-Alkyl und $R^2$ Wasserstoff bedeuten, reduziert, oder

f) zur Herstellung einer Verbindung der Formel I, worin $R^1$ und $R^2$ je Wasserstoff bedeuten, eine Verbindung der Formel I, worin $R^1$ Benzyl und $R^2$ Wasserstoff bedeuten, debenzyliert, und

g) erwünschtenfalls ein erhaltenes Racemat in die optischen Antipoden auftrennt, und/oder

h) erwünschtenfalls eine erhaltene Verbindung in ein pharmazeutisch verwendbares Säureadditionssalz überführt.

2. Verfahren gemäss Anspruch 1, worin $R^2$ Wasserstoff oder Acetyl bedeutet.

3. Verfahren gemäss Anspruch 2, worin $R^2$ Wasserstoff bedeutet.

4. Verfahren gemäss einem der Ansprüche 1-3, worin $R^1$ Wasserstoff, nieder-Alkyl, welches gegebenenfalls durch Hydroxy, Cyan oder substituiertes Benzoyl substituiert ist, Cycloalkyl, Cycloalkylalkyl oder gegebenenfalls durch Hydroxy substituiertes Aryl-nieder-alkyl bedeutet.

5. Verfahren gemäss Anspruch 4, worin $R^1$ Wasserstoff, nieder-Alkyl, welches gegebenenfalls durch Hydroxy, Cyan oder 4-Methoxybenzoyl substituiert ist, Cyclopentyl, Cyclopentyläthyl oder 4-Hydroxyphenäthyl bedeutet.

6. Verfahren gemäss Anspruch 5, worin es sich bei der nieder-Alkylgruppe um Aethyl, Propyl oder

Isopropyl handelt.

7. Verfahren gemäss einem der Ansprüche 1-6, worin $R^1$ Wasserstoff, gegebenenfalls durch Hydroxy, Cyan oder 4-Methoxybenzoyl substituiertes Aethyl, Propyl oder Isopropyl, Cyclopentyl, Cyclopentyläthyl oder 4-Hydroxyphenäthyl und $R^2$ Wasserstoff bedeuten.

8. Verfahren gemäss Anspruch 1, dadurch gekennzeichnet, dass man rac-1,2,3,4,4aα,5,6,10bβ-Octahydro-2α-methyl-4-propylbenzo[f]chinolin-7-ol, rac-1,2,3,4,4aα,5,6,10bβ-Octahydro-4-(4-hydroxyphenäthyl)-2α-methylbenzo[f]chinolin-7-ol, rac-1,2,3,4,4aα,5,6,10bβ-Octahydro-7-hydroxy-2α-methylbenzo[f]-chinolin-4-äthanol, rac-1,2,3,4,4aα,5,6,10bβ-Octahydro-7-hydroxy-2α-methylbenzo[f]chinolin-4-butyronitril, rac-1,2,3,4,4aα,5,6,10bβ-Octahydro-4-cyclopentyl-2α-methylbenzo[f]chinolin-7-ol, rac-1,2,3,4,4aα,5,6,10bβ-Octahydro-4-isopropyl-2α-methylbenzo[f]chinolin-7-ol, rac-1,2,3,4,4aα,5,6,10bβ-Octahydro-4-cyclopentyläthyl-2α-methylbenzo[f]chinolin-7-ol, rac-[1,2,3,4,4aα,5,6,10bβ-Octahydro-7-hydroxy-2α-methylbenzo[f]chinolin-4-yl]-4′-methoxybutyrophenon oder rac-1,2,3,4,4aα,5,6,10bβ-Octahydro-2α-methylbenzo[f]chinolin-7-ol herstellt.

9. Verfahren gemäss Anspruch 1, dadurch gekennzeichnet, dass man (-)-1,2,3,4,4aα,5,6,10bβ-Octahydro-4-cyclopentyl-2α-methylbenzo[f]chinolin-7-ol herstellt.

10. Verfahren gemäss Anspruch 1, dadurch gekennzeichnet, dass man (-)-1,2,3,4,4aα,5,6,10bβ-Octahydro-2α-methyl-4-propylbenzo[f]chinolin-7-ol herstellt.

11. Verfahren gemäss Anspruch 1, dadurch gekennzeichnet, dass man (-)-1,2,3,4,4aα,5,6,10bβ-Octahydro-7-hydroxy-2α-methylbenzo[f]chinolin-4-äthanol herstellt.

12. Verfahren gemäss Anspruch 1, dadurch gekennzeichnet, dass man (-)-1,2,3,4,4aα,5,6,10bβ-Octahydro-2α-methylbenzo[f]chinolin-7-ol herstellt.

13, Verfahren zur Herstellung von Arzneimitteln, insbesondere zur Verwendung bei der Bekämpfung bzw. Verhütung von psychotischen Störungen, insbesondee von chronischer Schizophrenie, dadurch gekennzeichnet, dass man ein Octahydrobenzo[f]chinolin der Formel I in Anspruch 1, in Form des Racemates oder eines optischen Antipoden, oder ein pharmazeutisch verwendbares Säureadditionssalz davon in eine galenische Darreichungsform bringt.

14. Verwendung eines Octahydrobenzo[f]chinolins der Formel I in Anspruch 1, in Form eines Racemates oder eines optischen Antipoden, oder eines pharmazeutisch verwendbaren Säureadditionssalzes davon zur Herstellung von Mitteln gegen psychotische Störungen, insbesondere gegen chronische Schizophrenie.